# EUROPEAN PATENT APPLICATION

(11) **EP 4 722 251 A1**
(43) Date of publication of application: **08.04.2026**
(21) Application number: 23940477.5
(22) Date of filing: 06.12.2023
(51) Int. Cl.: C07K 16/38, A61K 39/395, A61P 7/04

(54) **ANTI-TFPI ANTIBODY HAVING BINDING SPECIFICITY AND USE THEREOF**

(30) Priority: 05.06.2023 WO PCT/CN2023/098242
(71) Applicant: Ampsource Biopharma Shanghai Inc., Shanghai 201318 (CN)
(72) Inventor: LI, Qiang, Shanghai 201318 (CN); DIAO, Jiasheng, Shanghai 201318 (CN); SUN, Jianyu, Shanghai 201318 (CN); ZHOU, Chi, Shanghai 201318 (CN); WU, Cui, Shanghai 201318 (CN); GAO, Yongjuan, Shanghai 201318 (CN); LI, Yuanli, Shanghai 201318 (CN); CHEN, Si, Shanghai 201318 (CN)
(74) Representative: J A Kemp LLP
(86) International application number: PCT/CN2023/136763
(87) International publication number: WO 2024/250602

(57) **Abstract**

The present invention provides an antibody or an antigen-binding fragment thereof that specifically binds to an epitope shown in SEQ ID NO:1 in Tissue Factor Pathway Inhibitor (TFPI). It also provides a nucleic acid molecule encoding the antibody, an expression vector and a host cell for expressing the antibody, as well as a method for producing the antibody. Furthermore, the invention provides a pharmaceutical composition comprising the antibody or the antigen-binding fragment thereof, and its use in the preparation of a medicament for preventing and/or treating coagulation disorders in patients, such as hereditary or acquired coagulation factor deficiency, Bernard-Soulier Syndrome, and Glanzmann Thrombasthenia.

## Description

### Technical Field

The present invention belongs to the field of therapeutic monoclonal antibodies. More specifically, the invention relates to an antibody or an antigen-binding fragment thereof targeting Tissue Factor Pathway Inhibitor (TFPI); it also relates to the use of the antibody in the preparation of a medication for preventing or treating hereditary or acquired coagulation disorders or events.

### Background

Hemophilia A and B are hereditary bleeding disorders caused by coagulation dysfunction, characterized by impaired generation of active prothrombinase, prolonged coagulation time, and a lifelong tendency to bleed after minor trauma. Severe patients may experience spontaneous bleeding without obvious injury. Hemophilia A is characterized by the absence or deficiency of coagulation factor VIII (FVIII), accounting for over 80% of all hemophilia cases, with a prevalence of 1 in 5,000 males. Hemophilia B is characterized by the absence or deficiency of coagulation factor IX (FIX), with an incidence of 1 in 30,000 newborns (Chowdary, P. Int J Hematol, 2020, 111: 42-50). Recurrent, spontaneous, and trauma-related bleeding are characteristic of severe hemophilia, typical bleeding sites being joints and muscles. With minimal treatment, severe patients may experience up to 30-40 bleeding episodes per year, leading to irreversible joint damage and secondary disability. The most common current treatment is factor replacement therapy, supplementing the deficient FVIII or FIX in hemophilia patients. Based on current treatment methods, prophylactic therapy requires frequent infusions of FVIII or FIX, typically 2 to 4 times weekly, to reduce spontaneous bleeding. While this treatment can effectively prevent most spontaneous bleeds, it does not prevent traumatic bleeding. Furthermore, the effectiveness of treatment is influenced by infusion frequency, dose, and the number of missed or delayed doses, while the dose and infusion frequency are themselves constrained by the factor's elimination half-life, the cost-effectiveness of the regimen, and patient acceptability. To overcome the limitations of factor replacement therapy, improve patient compliance, and reduce patient burden, there is a need to explore new therapeutic approaches.

Platelets are a major component of the blood system. When blood vessels are damaged, platelets play a role in hemostasis and coagulation. A reduction in platelet count or impaired function leads to dysfunctional hemostasis and poor thrombus formation, causing related platelet disorders. Bernard-Soulier Syndrome, also known as giant platelet syndrome, is a rare hereditary bleeding disorder with an incidence of less than 1 per 1 million. It is primarily caused by defects in the synthesis and expression of the platelet membrane glycoprotein complex GPIb-IX-V, or by genetic defects in any of its subunits GPlb, GPIX, and GPV. The defect in GPIb-IX-V prevents platelets from properly adhering to the vascular endothelium, leading to skin, mucosal, or internal bleeding of varied severity. Large platelets and mild to moderate thrombocytopenia are observed in the patient's peripheral blood. Glanzmann thrombasthenia (GT) was first reported by Glanzmann in 1918, hence the name "Glanzmann's disease." It is a hereditary bleeding disorder characterized by hypo-responsiveness or lack of response of platelets to various physiological agonists, caused by qualitative or quantitative abnormalities of platelet membrane glycoprotein IIb (GPIIb) and/or IIIa (GPIIIa). In 1990, the Standardization Committee of the International Society on Thrombosis and Haemostasis defined GT as a congenital, hereditary condition of absent or markedly reduced platelet aggregation in response to multiple agonists (e.g., ADP, thrombin, collagen) due to defects in the genes encoding GPIIb or GPIIIa.

Tissue Factor Pathway Inhibitor (TFPI) is an endogenous natural anticoagulant protein that controls initiation phase of coagulation. It specifically inhibits the tissue factor-initiated coagulation pathway by binding to the TF/FVIIa complex, preventing factor X activation and thereby inhibiting coagulation function. When TFPI activity is neutralized by anti-TFPI antibodies, the coagulation process is restored or amplified. TFPI is a glycoprotein comprising 276 amino acid residues, containing three Kunitz domains (K1, K2, and K3), two linker regions, an acidic amino acid-rich N-terminus, and a basic amino acid-rich C-terminus. Domains K1 and K2 are critical for TFPI's anticoagulant activity; K3 is not essential for this role but its presence allows TFPI to better inhibit coagulation. Recent studies indicate that the K3 and C-terminal regions can bind to heparin, membrane surface glycoproteins, and polysaccharides, and are also involved in TFPI's anti-inflammatory effects. TFPI's inhibitory action in the coagulation pathway is achieved in two steps: first, TFPI binds to activated FX via K2 and competitively inhibits its activity, a Ca2+-independent reversible process; second, TFPI within the FXa/TFPI complex binds via K1 to the active site of FVIIa in the FVIIa/TF complex, thereby inhibiting the FVIIa/TF complex. Anti-TFPI antibodies act on the K2 region of TFPI, competitively blocking the binding site for FXa on TFPI, increasing FXa levels, and also blocking TFPI's inhibition of TF-FVIIa in the presence of FXa and Ca2+, leading to increased generation of FXa and thrombin, thereby enhancing coagulation capacity through a dual mechanism.

Anti-TFPI antibodies can be applied in hemophilia patients with or without inhibitors against FVIII and FIX, in Bernard-Soulier Syndrome, and in Glanzmann Thrombasthenia. The antibodies themselves have a longer half-life, potentially reducing dosing frequency. The field still needs to explore therapeutic antibodies with improved performance in terms of in vivo circulating half-life, in vivo stability, drug safety, and manufacturability.

### Details of the Invention

The present invention provides an antibody capable of specifically binding human TFPI with good safety window, the specific binding epitope of the antibody, nucleic acids encoding the antibody, vectors containing the nucleic acids, host cells containing the vectors, methods for producing the antibody, and pharmaceutical compositions for preventing or treating coagulation disorders in patients with hereditary or acquired coagulation factor deficiency. The pharmaceutical compositions contain the antibody as an active ingredient and are capable of inhibiting TFPI to activate the coagulation pathway. The disclosed anti-human TFPI antibodies can be used to treat or prevent hemophilia.

In one aspect, the invention provides an antibody or antigen-binding fragment thereof capable of specifically binding TFPI, wherein the antibody or antigen-binding fragment specifically binds to an epitope within residues K93 to N152 of the amino acid sequence shown in SEQ ID NO:1, and wherein the antibody or antigen-binding fragment does not bind to site R107 within 5 Å. Preferably, the antibody or antigen-binding fragment interacts with and/or blocks at least one of the following amino acid sites within the antigenic epitope at a distance of 5 Å or less: Y109, I110, T111, R124, K126, and E138. Preferably, the closest distance between the CA atom of an amino acid residue of the antibody or antigen-binding fragment and the CA atom of TFPI residue R107 is greater than 9.0 Å.

Preferably, when the antibody or antigen-binding fragment binds TFPI, in addition to interacting with and/or blocking at least one of amino acids Y109, I110, T111, R124, K126, or E138 at 5 Å or less, and not binding site R107, it also interacts with and/or blocks amino acids at at least 1, 2, 3, 4, 5, 6, 7, 8, 9, 10 or more of the following positions at 5 Å or less: T139, T119, E101, Y113, F114, N116, Q118, Q121, C122, E123, F125, and L140.

In certain aspects, when the antibody or antigen-binding fragment binds TFPI, in addition to interacting with and/or blocking 1 of amino acids Y109, I110, T111, R124, K126, or E138 at 5 Å or less, and not binding site R107, it also interacts with and/or blocks 1 of the following amino acids at 5 Å or less: T139, T119, E101, Y113, F114, N116, Q118, Q121, C122, E123, F125, and L140. In certain aspects, when the antibody or antigen-binding fragment binds TFPI, in addition to interacting with and/or blocking 2 of amino acids Y109, I110, T111, R124, K126, or E138 at 5 Å or less, and not binding site R107, it also interacts with and/or blocks 1 of the following amino acids at 5 Å or less: T139, T119, E101, Y113, F114, N116, Q118, Q121, C122, E123, F125, and L140. In certain aspects, when the antibody or antigen-binding fragment binds TFPI, in addition to interacting with and/or blocking 2 of amino acids Y109, I110, T111, R124, K126, or E138 at 5 Å or less, and not binding site R107, it also interacts with and/or blocks 2 of the following amino acids at 5 Å or less: T139, T119, E101, Y113, F114, N116, Q118, Q121, C122, E123, F125, and L140. In certain aspects, when the antibody or antigen-binding fragment binds TFPI, in addition to interacting with and/or blocking 3 of amino acids Y109, I110, T111, R124, K126, or E138 at 5 Å or less, and not binding site R107, it also interacts with and/or blocks 1 of the following amino acids at 5 Å or less: T139, T119, E101, Y113, F114, N116, Q118, Q121, C122, E123, F125, and L140.

Preferably, the antibody or antigen-binding fragment specifically binds to at least one or several basic amino acids in the TFPI protein, the basic amino acids selected from R112, K93, K120, R124, K126, and K144; preferably the basic amino acids are R124 and K126.

TFPI K2 R107 is a central amino acid involved in inhibiting the active center of FXa. In certain embodiments, the shortest distance between the CA atom of an amino acid residue of the antibody or antigen-binding fragment of the invention and the CA atom of TFPI K2 amino acid residue R107 is 10.50 Å, located between antibody light chain Y95 and TFPI K2 R107.

Preferably, the antibody or antigen-binding fragment comprises VH and VL, wherein the heavy chain variable region, as defined by the Kabat numbering system, comprises at least one, two, or three CDRs selected from the group consisting of:
(1) HCDR1 having the amino acid sequence SYWMH;
(2) HCDR2 having the amino acid sequence EIKPDX₁GRIX₂YNEKFKT, wherein X₁ is selected from Q, H, or K, and X₂ is selected from N, D, or H; and
(3) HCDR3 having the amino acid sequence SSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or, the light chain variable region comprises at least one, two, or three CDRs selected from the group consisting of:
(4) LCDR1 having the amino acid sequence SASSSVSSSYLY;
(5) LCDR2 having the amino acid sequence GTSILAS; and
(6) LCDR3 having the amino acid sequence HQWX₇X₈YPFT, wherein X₇ is selected from S or T, and X₈ is selected from H, Y, or F.

Preferably, the antibody or antigen-binding fragment comprises VH and VL, wherein the heavy chain variable region, as defined by the IMGT numbering system, comprises at least one, two, or three CDRs selected from the group consisting of:
(1) HCDR1 having the amino acid sequence GYTFTSYW;
(2) HCDR2 having the amino acid sequence IKPDX₁GRI, wherein X₁ is selected from Q, H, or K; and
(3) HCDR3 having the amino acid sequence ARSSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or, the light chain variable region comprises at least one, two, or three CDRs selected from the group consisting of:
(4) LCDR1 having the amino acid sequence SSVSSSY;
(5) LCDR2 having the amino acid sequence GTS; and
(6) LCDR3 having the amino acid sequence HQWX₇X₈YPFT, wherein X₇ is selected from S or T, and X₈ is selected from H, Y, or F.

In certain preferred embodiments, the HCDR1, HCDR2, and HCDR3 contained in the heavy chain variable region, and/or the LCDR1, LCDR2, and LCDR3 contained in the light chain variable region are defined by the Kabat or IMGT numbering system. Table 2 exemplarily provides the CDR amino acid sequences of preferred antibodies defined by the Kabat or IMGT numbering systems.

In certain preferred embodiments, the antibody or antigen-binding fragment comprises 3 VH CDRs and 3 VL CDRs as defined by the Kabat numbering system, selected from the group consisting of:
(1) HCDR1, HCDR2, HCDR3, LCDR1, LCDR2, and LCDR3 having the sequences as shown in SEQ ID NOs: 46, 47, 48, 49, 50, and 51, respectively, or sequences having one or several amino acid substitutions, deletions, or additions (e.g., 1, 2, or 3 substitutions, deletions, or additions) compared to any of the aforementioned sequences;
(2) HCDR1, HCDR2, HCDR3, LCDR1, LCDR2, and LCDR3 having the sequences as shown in SEQ ID NOs: 46, 47, 56, 49, 50, and 51, respectively, or sequences having one or several amino acid substitutions, deletions, or additions (e.g., 1, 2, or 3 substitutions, deletions, or additions) compared to any of the aforementioned sequences;
(3) HCDR1, HCDR2, HCDR3, LCDR1, LCDR2, and LCDR3 having the sequences as shown in SEQ ID NOs: 46, 47, 58, 49, 50, and 51, respectively, or sequences having one or several amino acid substitutions, deletions, or additions (e.g., 1, 2, or 3 substitutions, deletions, or additions) compared to any of the aforementioned sequences;
(4) HCDR1, HCDR2, HCDR3, LCDR1, LCDR2, and LCDR3 having the sequences as shown in SEQ ID NOs: 46, 60, 48, 49, 50, and 51, respectively, or sequences having one or several amino acid substitutions, deletions, or additions (e.g., 1, 2, or 3 substitutions, deletions, or additions) compared to any of the aforementioned sequences;
(5) HCDR1, HCDR2, HCDR3, LCDR1, LCDR2, and LCDR3 having the sequences as shown in SEQ ID NOs: 46, 61, 48, 49, 50, and 51, respectively, or sequences having one or several amino acid substitutions, deletions, or additions (e.g., 1, 2, or 3 substitutions, deletions, or additions) compared to any of the aforementioned sequences;
(6) HCDR1, HCDR2, HCDR3, LCDR1, LCDR2, and LCDR3 having the sequences as shown in SEQ ID NOs: 46, 63, 48, 49, 50, and 51, respectively, or sequences having one or several amino acid substitutions, deletions, or additions (e.g., 1, 2, or 3 substitutions, deletions, or additions) compared to any of the aforementioned sequences;
(7) HCDR1, HCDR2, HCDR3, LCDR1, LCDR2, and LCDR3 having the sequences as shown in SEQ ID NOs: 46, 65, 48, 49, 50, and 51, respectively, or sequences having one or several amino acid substitutions, deletions, or additions (e.g., 1, 2, or 3 substitutions, deletions, or additions) compared to any of the aforementioned sequences;
(8) HCDR1, HCDR2, HCDR3, LCDR1, LCDR2, and LCDR3 having the sequences as shown in SEQ ID NOs: 46, 47, 48, 49, 50, and 66, respectively, or sequences having one or several amino acid substitutions, deletions, or additions (e.g., 1, 2, or 3 substitutions, deletions, or additions) compared to any of the aforementioned sequences;
(9) HCDR1, HCDR2, HCDR3, LCDR1, LCDR2, and LCDR3 having the sequences as shown in SEQ ID NOs: 46, 47, 48, 49, 50, and 67, respectively, or sequences having one or several amino acid substitutions, deletions, or additions (e.g., 1, 2, or 3 substitutions, deletions, or additions) compared to any of the aforementioned sequences;
(10) HCDR1, HCDR2, HCDR3, LCDR1, LCDR2, and LCDR3 having the sequences as shown in SEQ ID NOs: 46, 47, 48, 49, 50, and 68, respectively, or sequences having one or several amino acid substitutions, deletions, or additions (e.g., 1, 2, or 3 substitutions, deletions, or additions) compared to any of the aforementioned sequences;
(11) HCDR1, HCDR2, HCDR3, LCDR1, LCDR2, and LCDR3 having the sequences as shown in SEQ ID NOs: 46, 63, 58, 49, 50, and 51, respectively, or sequences having one or several amino acid substitutions, deletions, or additions (e.g., 1, 2, or 3 substitutions, deletions, or additions) compared to any of the aforementioned sequences;
(12) HCDR1, HCDR2, HCDR3, LCDR1, LCDR2, and LCDR3 having the sequences as shown in SEQ ID NOs: 46, 47, 69, 49, 50, and 51, respectively, or sequences having one or several amino acid substitutions, deletions, or additions (e.g., 1, 2, or 3 substitutions, deletions, or additions) compared to any of the aforementioned sequences;
(13) HCDR1, HCDR2, HCDR3, LCDR1, LCDR2, and LCDR3 having the sequences as shown in SEQ ID NOs: 46, 47, 71, 49, 50, and 51, respectively, or sequences having one or several amino acid substitutions, deletions, or additions (e.g., 1, 2, or 3 substitutions, deletions, or additions) compared to any of the aforementioned sequences;
(14) HCDR1, HCDR2, HCDR3, LCDR1, LCDR2, and LCDR3 having the sequences as shown in SEQ ID NOs: 46, 47, 73, 49, 50, and 51, respectively, or sequences having one or several amino acid substitutions, deletions, or additions (e.g., 1, 2, or 3 substitutions, deletions, or additions) compared to any of the aforementioned sequences;
(15) HCDR1, HCDR2, HCDR3, LCDR1, LCDR2, and LCDR3 having the sequences as shown in SEQ ID NOs: 46, 47, 75, 49, 50, and 51, respectively, or sequences having one or several amino acid substitutions, deletions, or additions (e.g., 1, 2, or 3 substitutions, deletions, or additions) compared to any of the aforementioned sequences;
(16) HCDR1, HCDR2, HCDR3, LCDR1, LCDR2, and LCDR3 having the sequences as shown in SEQ ID NOs: 46, 47, 77, 49, 50, and 51, respectively, or sequences having one or several amino acid substitutions, deletions, or additions (e.g., 1, 2, or 3 substitutions, deletions, or additions) compared to any of the aforementioned sequences;
(17) HCDR1, HCDR2, HCDR3, LCDR1, LCDR2, and LCDR3 having the sequences as shown in SEQ ID NOs: 46, 47, 79, 49, 50, and 51, respectively, or sequences having one or several amino acid substitutions, deletions, or additions (e.g., 1, 2, or 3 substitutions, deletions, or additions) compared to any of the aforementioned sequences;
(18) HCDR1, HCDR2, HCDR3, LCDR1, LCDR2, and LCDR3 having the sequences as shown in SEQ ID NOs: 46, 47, 81, 49, 50, and 51, respectively, or sequences having one or several amino acid substitutions, deletions, or additions (e.g., 1, 2, or 3 substitutions, deletions, or additions) compared to any of the aforementioned sequences;
(19) HCDR1, HCDR2, HCDR3, LCDR1, LCDR2, and LCDR3 having the sequences as shown in SEQ ID NOs: 46, 47, 83, 49, 50, and 51, respectively, or sequences having one or several amino acid substitutions, deletions, or additions (e.g., 1, 2, or 3 substitutions, deletions, or additions) compared to any of the aforementioned sequences;
(20) HCDR1, HCDR2, HCDR3, LCDR1, LCDR2, and LCDR3 having the sequences as shown in SEQ ID NOs: 46, 47, 85, 49, 50, and 51, respectively, or sequences having one or several amino acid substitutions, deletions, or additions (e.g., 1, 2, or 3 substitutions, deletions, or additions) compared to any of the aforementioned sequences;
(21) HCDR1, HCDR2, HCDR3, LCDR1, LCDR2, and LCDR3 having the sequences as shown in SEQ ID NOs: 46, 47, 87, 49, 50, and 51, respectively, or sequences having one or several amino acid substitutions, deletions, or additions (e.g., 1, 2, or 3 substitutions, deletions, or additions) compared to any of the aforementioned sequences;
(22) HCDR1, HCDR2, HCDR3, LCDR1, LCDR2, and LCDR3 having the sequences as shown in SEQ ID NOs: 46, 47, 89, 49, 50, and 51, respectively, or sequences having one or several amino acid substitutions, deletions, or additions (e.g., 1, 2, or 3 substitutions, deletions, or additions) compared to any of the aforementioned sequences.

In certain preferred embodiments, the substitutions in any of the above items are conservative substitutions.

In certain preferred embodiments, the antibody or antigen-binding fragment comprises 3 VH CDRs and 3 VL CDRs as defined by the IMGT numbering system, selected from the group consisting of:
(1) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(2) HCDR1, HCDR2, HCDR3, LCDR1, LCDR2, and LCDR3 having the sequences as shown in SEQ ID NOs: 52, 53, 57, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(3) HCDR1, HCDR2, HCDR3, LCDR1, LCDR2, and LCDR3 having the sequences as shown in SEQ ID NOs: 52, 53, 59, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(4) HCDR1, HCDR2, HCDR3, LCDR1, LCDR2, and LCDR3 having the sequences as shown in SEQ ID NOs: 52, 62, 54, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(5) HCDR1, HCDR2, HCDR3, LCDR1, LCDR2, and LCDR3 having the sequences as shown in SEQ ID NOs: 52, 65, 54, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(6) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 66, respectively, or sequences having one or several amino acid substitutions, deletions, or additions (e.g., 1, 2, or 3 substitutions, deletions, or additions) compared to any of the aforementioned sequences;
(7) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 67, respectively, or sequences having one or several amino acid substitutions, deletions, or additions (e.g., 1, 2, or 3 substitutions, deletions, or additions) compared to any of the aforementioned sequences;
(8) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 68, respectively, or sequences having one or several amino acid substitutions, deletions, or additions (e.g., 1, 2, or 3 substitutions, deletions, or additions) compared to any of the aforementioned sequences;
(9) HCDR1, HCDR2, HCDR3, LCDR1, LCDR2, and LCDR3 having the sequences as shown in SEQ ID NOs: 52, 64, 59, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(10) HCDR1, HCDR2, HCDR3, LCDR1, LCDR2, and LCDR3 having the sequences as shown in SEQ ID NOs: 52, 53, 70, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(11) HCDR1, HCDR2, HCDR3, LCDR1, LCDR2, and LCDR3 having the sequences as shown in SEQ ID NOs: 52, 53, 72, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(12) HCDR1, HCDR2, HCDR3, LCDR1, LCDR2, and LCDR3 having the sequences as shown in SEQ ID NOs: 52, 53, 74, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(13) HCDR1, HCDR2, HCDR3, LCDR1, LCDR2, and LCDR3 having the sequences as shown in SEQ ID NOs: 52, 53, 76, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(14) HCDR1, HCDR2, HCDR3, LCDR1, LCDR2, and LCDR3 having the sequences as shown in SEQ ID NOs: 52, 53, 78, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(15) HCDR1, HCDR2, HCDR3, LCDR1, LCDR2, and LCDR3 having the sequences as shown in SEQ ID NOs: 52, 53, 80, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(16) HCDR1, HCDR2, HCDR3, LCDR1, LCDR2, and LCDR3 having the sequences as shown in SEQ ID NOs: 52, 53, 82, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(17) HCDR1, HCDR2, HCDR3, LCDR1, LCDR2, and LCDR3 having the sequences as shown in SEQ ID NOs: 52, 53, 84, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(18) HCDR1, HCDR2, HCDR3, LCDR1, LCDR2, and LCDR3 having the sequences as shown in SEQ ID NOs: 52, 53, 86, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(19) HCDR1, HCDR2, HCDR3, LCDR1, LCDR2, and LCDR3 having the sequences as shown in SEQ ID NOs: 52, 53, 88, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(20) HCDR1, HCDR2, HCDR3, LCDR1, LCDR2, and LCDR3 having the sequences as shown in SEQ ID NOs: 52, 53, 90, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences.

In certain preferred embodiments, the substitutions in any of the above items are conservative substitutions.

In certain embodiments, the antibody or antigen-binding fragment is humanized. Example 3 provides a basic workflow of the humanization strategy, and Table 3 lists the amino acid sequence numbers of the variable regions of preferred humanized antibodies.

In certain preferred embodiments, the humanized antibody or antigen-binding fragment comprises the following VH and VL sequences: a VH domain comprising the amino acid sequence as shown in SEQ ID NO:2, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:3, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:4, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:5, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:6, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:7, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:8, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:9, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:10, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:11, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:12, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:13, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:14, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:15, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:16, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:17, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:18, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:19, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:20, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:21, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:22, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:23, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:24, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:25, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:26, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:27, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:28, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:29, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:30, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:31, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:32, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:33, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:34, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:35, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:36, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:37, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:38, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:39, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:40, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:41, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:42, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:43, or a sequence substantially identical thereto (e.g., at least 80%, 85%, 90%, 92%, 95%, 97%, 98%, 99% or higher identity, or having one or more amino acid substitutions (e.g., conservative substitutions)).

In certain preferred embodiments, the humanized antibody or antigen-binding fragment comprises the following VH and VL sequences: a VH domain comprising the amino acid sequence as shown in SEQ ID NO:44, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:45, or a sequence substantially identical thereto (e.g., at least 80%, 85%, 90%, 92%, 95%, 97%, 98%, 99% or higher identity, or having one or more amino acid substitutions (e.g., conservative substitutions)).

In certain embodiments, the antibody or antigen-binding fragment is a full-length antibody, Fab, Fab', (Fab')2, Fd, Fv, dAb, scFv, or scFv-scFv.

In certain embodiments, the antibody comprises a heavy chain constant region and a light chain constant region derived from human immunoglobulins.

Preferably, the antibody comprises the amino acid sequence of a human kappa light chain constant region.

Preferably, the antibody comprises a heavy chain constant region selected from human IgG1, IgG2, IgG3, IgG4, IgM, IgA1, IgA2, IgD, and IgE; more preferably, selected from human IgG1, IgG2, and IgG4; and, the heavy chain constant region has a natural sequence or a sequence having one or more amino acid substitutions, deletions, or additions compared to the natural sequence from which it is derived.

Preferably, the antibody comprises a heavy chain constant region of human IgG4, and the heavy chain constant region contains the amino acid mutation S228P.

In any of the above embodiments, the antibody or antigen-binding fragment of the invention is capable of binding TFPI with a K_{D} of 10 nM or lower, more preferably with a K_{D} of 1 nM or lower; more preferably with a K_{D} of 100 pM or lower; even more preferably with a K_{D} of 10 pM or lower.

In one aspect, the invention provides a bispecific antibody or antigen-binding portion thereof, comprising the antibody or antigen-binding portion thereof described above linked to a second antibody or antigen-binding portion thereof. It is characterized in that the second antibody or antigen-binding portion binds to TFPI.

In one aspect, the invention provides a DNA molecule encoding the antibody or antigen-binding fragment described above.

In one aspect, the invention provides a vector comprising the DNA molecule described above.

In one aspect, the invention provides a host cell comprising the vector described above; the host cell comprises prokaryotic cells, yeast, or mammalian cells, such as CHO cells, NS0 cells, or other mammalian cells, preferably CHO cells.

In one aspect, the invention provides a pharmaceutical composition comprising the antibody or antigen-binding fragment described above, along with a pharmaceutically acceptable excipient, carrier, or diluent.

In certain embodiments, the pharmaceutical composition can be administered to a patient via subcutaneous injection. For example, a dose of 10 to 500 mg of the anti-TFPI antibody can be administered to a patient via subcutaneous injection at a frequency of once daily, every 2 days, every 3 days, every 4 days, every 5 days, every 6 days, twice weekly, once weekly, once every two weeks, or once monthly.

In one aspect, the invention provides the use of the antibody or antigen-binding fragment in the manufacture of a medicament for preventing or treating a disease or event associated with hereditary or acquired coagulation factor deficiency. For example, the antibody or antigen-binding fragment provided by the invention can be used to interfere with the mutual influence between TFPI and FXa, or to prevent TFPI-dependent inhibition of TF/FVIIa activity. Furthermore, the antibody can also be used to restore TF/FVIIa-driven FXa generation, bypassing insufficient FXa amplification dependent on FVIII or FIX. Further, the diseases include hemophilia A and hemophilia B, and also encompass spontaneous bleeding events in hemophilia patients, traumatic bleeding events, or bleeding events occurring during surgical prophylaxis, perioperative management, or surgical treatment.

In other aspects, the invention provides a method for preventing or treating a disease or bleeding event associated with hereditary or acquired coagulation factor deficiency, the method comprising administering an effective amount of the antibody or antigen-binding fragment of the invention, or the pharmaceutical composition of the invention.

In one aspect, the invention provides the use of the antibody or antigen-binding fragment in the manufacture of a medicament for preventing or treating hereditary coagulation disorders. Preferably, the hereditary coagulation disorders are Glanzmann Thrombasthenia and Bernard-Soulier Syndrome.

The technical solutions provided by the invention have achieved beneficial effects, summarized as follows:
(1) The TFPI antibodies provided by the invention have good binding affinity, can specifically recognize human TFPI, and have efficacy for treating and preventing hemophilia, as preliminarily validated in clinical studies;
(2) The TFPI antibodies provided by the invention do not directly interact with the key amino acid R107 in the K2 domain of the TFPI protein. Instead, they achieve shielding of the nearby R107 site by directly binding to the adjacent Y109. Amino acid R107 in the K2 domain directly participates in inhibiting coagulation factor FXa and is a central position in an active region. Superimposition of the modeled structures of the AB8D/TFPI K2 and FXa/TFPI K2 complexes, aligned via the common TFPI K2 domain, indicates that the AB8D antibody has spatial blocking or steric hindrance with coagulation factor FXa, demonstrating that AB8D competes with FXa for binding to TFPI K2. Due to the high affinity of the AB8D antibody, it blocks the interaction between TFPI K2 and coagulation factor FXa via Y109 adjacent to R107. Within the effective dose range, the amplification factor of the coagulation cascade reaction is less prone to being super-amplified, thereby relatively reducing the risk of thrombosis and resulting in higher safety window.
(3) In preclinical repeated-dose toxicity studies and maximum tolerated dose studies, the TFPI antibodies of the invention showed no significant impact on the central nervous, cardiovascular, and respiratory systems of rhesus monkeys, no local irritation at the injection site, and no abnormal changes in immunotoxicity evaluation indicators related to the antibodies of the invention, demonstrating good safety and significant clinical value.

### Abbreviations and Definition of Terms

The following abbreviations are used herein:
CDR: Complementarity-Determining Region, within the variable region of an immunoglobulin, defined using the Kabat, IMGT, Chothia, or AbM numbering systems (see terms "hypervariable region" or "CDR region" or "complementarity determining region").
EC₅₀: Concentration producing 50% efficacy or binding
ELISA: Enzyme-Linked Immunosorbent Assay
FR: Framework Region of an antibody, the immunoglobulin variable region excluding the CDR regions
HRP: Horseradish Peroxidase
IC₅₀: Concentration producing 50% inhibition
IgG: Immunoglobulin G
Kabat: The immunoglobulin amino acid sequence alignment and numbering system advocated by Elvin A. Kabat.
PCR: Polymerase Chain Reaction
V region: Region of an IgG chain with variable sequence among different antibodies, extending to Kabat residue 109 in the light chain and residue 113 in the heavy chain.
K_{D}: Equilibrium dissociation constant
ka: Association rate constant
kd: Dissociation rate constant

In the present invention, unless otherwise specified, scientific and technical terms used herein have the meanings commonly understood by those skilled in the art. Furthermore, laboratory procedures involving cell culture, biochemistry, nucleic acid chemistry, immunology, etc., as used herein, are conventional steps widely employed in their respective fields. To better understand the invention, definitions and explanations of related terms are provided below.

The term "Tissue Factor Pathway Inhibitor" or "TFPI" refers to any variant, isoform, and species homolog of human TFPI naturally expressed by cells.

The term "EU numbering system" refers to the numbering scheme based on the amino acid sequence of the first human IgG1 immunoglobulin, designated "Eu," which was isolated and purified by Gerald M. Edelman et al. in the late 1960s (1968-1969), and for which the amino acid sequence was determined and numbered (Edelman GM et al, 1969, Proc Natl Acad USA, 63:78-85). The amino acid sequences of the heavy chain constant regions of other immunoglobulins are aligned with that of Eu, and the corresponding amino acid positions are assigned according to this numbering. The EU numbering system primarily applies to the heavy chain constant regions of immunoglobulins, including CH1, CH2, CH3, and the hinge region.

The term "Kabat numbering system" refers to the standardized numbering scheme for human immunoglobulin variable regions first proposed by Kabat et al. in 1979 (Kabat EA, Wu TT, Bilofsky H, Sequences of Immunoglobulin Chains: Tabulation and Analysis of Amino Acid Sequences of Precursors, V-regions, C-regions, J-Chain and β2-Microglobulins. 1979. Department of Health, Education, and Welfare, Public Health Service, National Institutes of Health). In the publication "Sequences of Proteins of Immunological Interest" (Kabat EA, Wu TT, Perry HM, Gottesman KS, Foeller C. 1991. Sequences of Proteins of Immunological Interest, 5th edition. Bethesda, MD: US Department of Health and Human Services, National Institutes for Health), Kabat et al. aligned and numbered the amino acid sequences of antibody light and heavy chains. They observed that these analyzed sequences exhibited variable lengths, and that deletions or insertions of amino acids or amino acid fragments could only occur at specific positions. Interestingly, insertion sites are often located within the CDRs but may also appear at certain positions in the framework regions. In the Kabat numbering scheme, the light chain variable region is numbered up to position 109, and the heavy chain variable region up to position 113. Inserted amino acids in the light and heavy chains are identified and annotated with letters (e.g., 27a, 27b, ...). All lambda light chains lack a residue at position 10, and lambda and kappa light chains are encoded by different genes located on different chromosomes. Lambda and kappa light chains can be distinguished by differences in their constant region amino acid sequences. Unlike the EU numbering system, which applies only to heavy chain constant regions, the Kabat numbering system covers the full-length immunoglobulin sequence, including the variable and constant regions of both immunoglobulin light and heavy chains.

The term "antibody" generally refers to a protein binding molecule having the function of an immunoglobulin class. Typical examples are immunoglobulins, as well as derivatives or functional fragments thereof, as long as they exhibit the desired binding specificity. Techniques for producing antibodies are well known in the art. "Antibody" includes different classes of natural immunoglobulins (e.g., IgA, IgG, IgM, IgD, and IgE) and subclasses (e.g., IgG1, IgG2, IgA1, IgA2, etc.). "Antibody" also includes non-natural immunoglobulins, including for example single-chain antibodies, chimeric antibodies (e.g., humanized murine antibodies), and heteroconjugate antibodies (e.g., bispecific antibodies), as well as antigen-binding fragments thereof (e.g., Fab', F(ab')2, Fab, Fv, and rIgG). See also, e.g., Pierce Catalog and Handbook, 1994-1995 (Pierce Chemical Co, Rockford, Ill); Kuby J, Immunology, 3rd Ed, WH Freeman & Co, New York, 1997. An antibody can bind to one antigen, termed "monospecific"; or bind to two different antigens, termed "bispecific"; or bind to more than one different antigen, termed "multispecific". An antibody can be monovalent, bivalent, or multivalent, i.e., an antibody can bind to one, two, or more antigen molecules at a time. An antibody binds "monovalent" to a particular protein, meaning one molecule of antibody binds to only one molecule of the protein, but the antibody may also bind to different proteins. When an antibody binds to only one molecule of each of two different proteins, the antibody binds "monovalent" to each protein, and the antibody is "bispecific" and binds "monovalent" to each of the two different proteins. An antibody can be "monomeric", i.e., it comprises a single polypeptide chain. An antibody may comprise multiple polypeptide chains ("multimeric") or may comprise two ("dimeric"), three ("trimeric"), or four ("tetrameric") polypeptide chains. If an antibody is multimeric, it may be a homomultimer, i.e., the antibody comprises more than one molecule of only one type of polypeptide chain, including homodimers, homotrimers, or homotetramers. Alternatively, a multimeric antibody may be a heteromultimer, i.e., the antibody comprises more than one type of different polypeptide chain, including heterodimers, heterotrimers, or heterotetramers.

The term "monoclonal antibody (mAb)" refers to an antibody obtained from a substantially homogeneous population of antibodies, e.g., the population contains individual antibodies that are identical except for possible naturally occurring mutations that may be present in minor amounts. Therefore, the modifier "monoclonal" indicates that the antibody is characterized not being a mixture of discrete antibodies. Monoclonal antibodies are produced by methods known to those skilled in the art, such as by fusing myeloma cells with immune spleen cells to prepare hybrid antibody-producing cells. They are synthesized by hybridoma culture and are not contaminated by other immunoglobulins. Monoclonal antibodies can also be obtained using techniques such as recombinant technology, phage display, synthetic technology, or other existing techniques.

The term "antigenic epitope" refers to any determinant capable of being bound by an antigen-binding protein, such as an antibody or T-cell receptor. An epitope is the region of an antigen bound by an antigen-binding protein targeting that antigen, and when the antigen is a protein, the epitope includes the specific amino acids that directly contact the antigen-binding protein. Most commonly, epitopes are located on proteins, but in some cases can be located on other kinds of molecules, such as nucleic acids. Epitope determinants can include chemically active surface groups of molecules such as amino acids, sugar side chains, phosphoryl or sulfonyl groups, and can have specific three-dimensional structural features and/or specific charge characteristics. Typically, an antibody specific for a particular target antigen will preferentially recognize an epitope on the target antigen within a complex mixture of proteins and/or macromolecules. An antigenic epitope can consist of a continuous sequence (primary structure of a protein) or be formed by a discontinuous protein three-dimensional structure. Most antigenic determinants exist on the surface of the antigenic substance. A natural antigenic substance can have multiple and numerous determinants. The larger the antigen molecule, the greater the number of determinants.

The term "binding" refers to, at the microscopic level, the binding between an antibody or its antigen-binding fragment and its corresponding target site, which can generally be considered as the corresponding amino acid residues of the two interacting and/or blocking each other at a distance of 5 Å or less. The interaction forces constituting the interaction and/or occlusion can include hydrogen bonds, van der Waals forces, electrostatic forces, cation-pi interactions, and/or hydrophobic interactions.

An antibody that "preferentially binds" or "specifically binds" (used interchangeably in this invention) to an epitope is a term well known in the art, and methods for determining such specificity or preferential binding are also well known. A molecule is said to exhibit "specific binding" or "preferential binding" if it reacts or associates more frequently, more rapidly, with greater duration, and/or with greater affinity with a particular cell or substance than it does with alternative cells or substances. If an antibody binds to a target with greater affinity, avidity, more readily, and/or with greater duration than it binds to other substances, it is said to "specifically bind" or "preferentially bind" to the target. Furthermore, if an antibody binds to a target in a sample with greater affinity, avidity, more readily, and/or with greater duration than it binds to other substances present in the sample, it is said to "specifically bind" or "preferentially bind" to the target in the sample. For example, an antibody that specifically or preferentially binds to a TFPI epitope is one that binds to that epitope with greater affinity, avidity, more readily, and/or with greater duration than it binds to other TFPI epitopes or non-TFPI epitopes. It is understood by reading this definition that, for example, an antibody (or portion or epitope thereof) that "specifically binds" or "preferentially binds" a first target may or may not specifically or preferentially bind a second target. Thus, "specific binding" or "preferential binding" does not necessarily require (although it may include) exclusive binding. Generally, but not necessarily, reference to binding means preferential binding. "Specific binding" or "preferential binding" includes compounds, such as proteins, nucleic acids, antibodies, etc., that can recognize and bind a specific molecule in a sample but do not substantially recognize or bind other molecules in the sample. For example, an antibody or peptide receptor that recognizes and binds its cognate ligand or binding partner (e.g., an anti-TFPI antibody binding TFPI) in a sample but does not substantially recognize or bind other molecules in the sample specifically binds that cognate ligand or binding partner. Thus, under designated assay conditions, the specific binding moiety (e.g., antibody or antigen-binding portion thereof or receptor or ligand-binding portion thereof) preferentially binds a particular target molecule and does not bind in significant amounts to other components present in the test sample.

The term "pKa" refers to the negative logarithm of the acid dissociation constant of an acidic substance. The pKa value is obtained by taking the negative base-10 logarithm of the acid dissociation constant Ka value, i.e., pKa = -log10(Ka). It is an indicator used to measure the strength of an acid. The smaller the pKa value, the stronger the acid; the larger the pKa value, the weaker the acid. The acid dissociation constant (Ka) refers to the equilibrium constant for the reaction in which an acid dissociates in water to generate hydrogen ions (H+) and the corresponding anion (A-). A larger Ka value indicates a stronger acid, meaning a higher degree of dissociation of the acid. HA 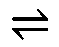 H+ + A-, Ka=[H+][A-]/[HA], where [HA], [H+], and [A-] are the concentrations of acid HA, hydrogen ion H+, and conjugate base A-, respectively.

The term "antibody fragment" or "antigen-binding fragment" refers to an antigen-binding fragment of an antibody and antibody analogs that retain the ability to specifically bind an antigen, typically comprising at least a portion of the antigen-binding region or variable region of the parental antibody. An antibody fragment retains at least some of the binding specificity of the parental antibody. Typically, when activity is expressed in molar units (KD), an antibody fragment retains at least 10% of the parental binding activity. Preferably, the antibody fragment retains at least 20%, 50%, 70%, 80%, 90%, 95%, or 100% of the binding affinity of the parental antibody for the target. Antibody fragments include, but are not limited to: Fab fragments, Fab' fragments, F(ab')2 fragments, Fv fragments, Fd fragments, complementarity determining region (CDR) fragments, disulfide-stabilized proteins (dsFv), etc.; linear antibodies, single-chain antibodies (e.g., scFv), unibodies (technology from Genmab), bivalent single-chain antibodies, single-chain phage antibodies, single-domain antibodies (e.g., VH domain antibodies), domain antibodies (technology from Domantis), nanobodies (technology from Ablynx); multispecific antibodies formed from antibody fragments (e.g., triabodies, tetrabodies, etc.); and engineered antibodies such as chimeric antibodies (e.g., humanized murine antibodies), heteroconjugate antibodies, etc. These antibody fragments are obtained using conventional techniques known to those skilled in the art and screened for utility using the same methods as for intact antibodies.

The term "single-chain Fv antibody" (or "scFv antibody") refers to an antibody fragment comprising the VH and VL domains of an antibody, a recombinant protein in which the heavy chain variable region (VH) and light chain variable region (VL) are connected by a linker, enabling these two domains to associate to form an antigen-binding site. The linker sequence typically consists of flexible peptides, for example but not limited to G4S(GGGGS)3. An scFv is generally about 1/6 the size of a full-length antibody. A single-chain antibody is preferably a single amino acid chain sequence encoded by one nucleotide chain. For reviews on scFv, see Pluckthun A, 1994. Antibodies from Escherichia coli, in The Pharmacology of Monoclonal Antibodies, Vol 113, Rosenberg M and Moore GP (EDs.), Springer-Verlag, New York, pp 269-315. See also International Patent Application Publication No. WO 88/01649 and U.S. Pat. Nos. 4,946,778 and 5,260,203.

The term "VL domain" refers to the amino-terminal variable region domain of an immunoglobulin light chain.

The term "VH domain" refers to the amino-terminal variable region domain of an immunoglobulin heavy chain.

The term "hinge region" includes that portion of the heavy chain molecule that connects the CH1 domain to the CH2 domain. This hinge region comprises about 25 residues and is flexible, allowing the two N-terminal antigen-binding regions to move independently. The hinge region can be divided into three distinct domains: upper, middle, and lower hinge domains (Roux KH et al, 1998, J Immunol, 161:4083-4090).

The term "Fv region" comprises variable regions from both heavy and light chains but lacks constant regions and is the smallest fragment containing a complete antigen recognition and binding site.

The term "heavy chain constant region" includes amino acid sequences from an immunoglobulin heavy chain. A polypeptide comprising a heavy chain constant region comprises at least one of the following: a CH1 domain, a hinge (e.g., upper hinge region, middle hinge region, and/or lower hinge region) domain, a CH2 domain, a CH3 domain, or a variant or fragment thereof. For example, an antigen-binding polypeptide used in this application may comprise a polypeptide chain having a CH1 domain; a polypeptide having a CH1 domain, at least a portion of a hinge domain, and a CH2 domain; a polypeptide chain having a CH1 domain and a CH3 domain; a polypeptide chain having a CH1 domain, at least a portion of a hinge domain, and a CH3 domain; or a polypeptide chain having a CH1 domain, at least a portion of a hinge domain, a CH2 domain, and a CH3 domain. In another embodiment, a polypeptide of the application comprises a polypeptide chain having a CH3 domain. Additionally, antibodies used in this application may lack at least a portion of the CH2 domain (e.g., all or a portion of the CH2 domain). As mentioned above, those of ordinary skill in the art will understand that the heavy chain constant region may be modified such that they differ in amino acid sequence from naturally occurring immunoglobulin molecules.

The term "light chain constant region" encompasses amino acid sequences derived from an antibody light chain. Preferably, the light chain constant region comprises at least one of a constant kappa domain and a constant lambda domain.

The term "Fc region" or "Fc fragment" refers to the C-terminal region of an immunoglobulin heavy chain, which contains at least a portion of the hinge region, the CH2 domain, and the CH3 domain. It mediates the binding of immunoglobulins to host tissues or factors, including binding to Fc receptors located on various cells of the immune system (e.g., effector cells) or to the first component (C1q) of the classical complement system. The Fc region includes native sequence Fc regions and variant Fc regions.

Typically, the human IgG heavy chain Fc region extends from its amino acid residue at Cys 226 or Pro 230 to the carboxyl terminus, but the boundaries may vary. The C-terminal lysine of the Fc region (residue 447, according to EU numbering) may be present or absent. Fc can also refer to this region when it exists independently or in the context of a protein polypeptide comprising the Fc, such as "a binding protein comprising an Fc region", also called an "Fc fusion protein" (e.g., an antibody or an immune-adhesin). The native sequence Fc region in the antibodies of the invention is derived from IgG1, IgG2 (IgG2A, IgG2B), IgG3, and IgG4, including mammalian (e.g., human) sources. In certain embodiments, relative to the mammalian Fc polypeptide amino acid sequence, the amino acid sequences of the two Fc polypeptide chains have approximately 10 single amino acid substitutions, insertions, and/or deletions per 100 amino acids. In some embodiments, the aforementioned Fc region amino acid differences may be Fc alterations that extend half-life, enhance FcRn binding, enhance Fcγ receptor (FcγR) binding, and/or enhance ADCC, ADCP, and/or CDC.

As used herein, the term "immune response" refers to the action of immune cells (e.g., lymphocytes, antigen-presenting cells, phagocytes, or granulocytes) and soluble macromolecules (including antibodies, cytokines, and complement) produced by immune cells or the liver, resulting in selective damage, destruction, or clearance of invasive pathogens, cells or tissues infected by pathogens, cancer cells, or in cases of autoimmunity or pathological inflammation, normal human cells or tissues from the human body. In the present invention, the term "antigen-specific T cell response" refers to an immune response produced by T cells when the T cells are stimulated by an antigen specific to them. Non-limiting examples of responses produced by T cells upon antigen-specific stimulation include T cell proliferation and production of cytokines (e.g., IL-2).

As used herein, the term "antibody-dependent cell-mediated cytotoxicity (ADCC)" refers to a form of cytotoxicity in which the Fc region of an antibody binds to Fc receptors (FcR) present on cytotoxic cells (e.g., natural killer (NK) cells, neutrophils, or macrophages), enabling these cytotoxic effector cells to specifically bind to target cells coated with the antigen, and then kill the target cells by secreting cytotoxins. Methods for detecting ADCC activity of antibodies are known in the art, for example, by assessing the binding activity between the test antibody and an Fc receptor (e.g., CD16a).

As used herein, the term "complement-dependent cytotoxicity (CDC)" refers to a form of cytotoxicity involving activation of the complement cascade via binding of the complement component C1q to the antibody Fc. Methods for detecting CDC activity of antibodies are known in the art, for example, by assessing the binding activity between the test antibody and an Fc receptor (e.g., C1q).

In the IgG, IgA, and IgD antibody isotypes, the Fc region comprises the CH2 and CH3 constant domains of each of the two heavy chains of the antibody; the IgM and IgE Fc regions comprise three heavy chain constant domains (CH2-4 domains) in each polypeptide chain.

The term "humanized antibody" refers to a genetically engineered non-human antibody whose amino acid sequence has been modified to increase homology with human antibody sequences. Most or all of the amino acids outside the CDR domains of the non-human antibody, e.g., a murine antibody, are replaced with corresponding amino acids from human immunoglobulins, while most or all of the amino acids within one or more CDR regions remain unchanged. Addition, deletion, insertion, substitution, or modification of amino acids is permitted as long as they do not eliminate the antibody's ability to bind a specific antigen. A "humanized" antibody retains antigen specificity similar to the original antibody. The source of the CDRs is not particularly limited and can be from any animal. For example, CDR regions derived from mouse, rat, rabbit, or non-human primate (e.g., cynomolgus monkey) antibodies can be utilized. Framework regions can be obtained by searching the IMGT antibody germline database (http://www.imgt.org/3Dstructure-DB/cgi/DomainGapAlign.cgi) for human antibody germline sequences. Generally, human germline antibody sequences with high homology to the non-human antibody being engineered are selected as the framework regions for the humanized antibody.

The term "hypervariable region" or "CDR region" or "complementarity determining region" refers to the amino acid residues of an antibody responsible for antigen binding, which are non-contiguous amino acid sequences. CDR sequences can be defined by the Kabat, Chothia, IMGT (Lefranc et al, 2003, Dev Comparat Immunol, 27:55-77) methods or by any CDR sequence determination method well known in the art. For example, hypervariable regions include amino acid residues from the "complementarity determining regions" or "CDRs" as defined by sequence alignment (Kabat numbering system), e.g., residues 24-34 (LCDR1), 50-56 (LCDR2), and 89-97 (LCDR3) in the light chain variable domain and residues 31-35 (HCDR1), 50-65 (HCDR2), and 95-102 (HCDR3) in the heavy chain variable domain. See Kabat et al, 1991, Sequences of Proteins of Immunological Interest, 5th Edition, Public Health Service, National Institutes of Health, Bethesda, Md.; and/or residues from the "hypervariable loops" (HVLS) as structurally defined (Chothia numbering system), e.g., residues 26-32 (LCDR1), 50-52 (LCDR2), and 91-96 (LCDR3) in the light chain variable domain and residues 26-32 (HCDR1), 53-55 (HCDR2), and 96-101 (HCDR3) in the heavy chain variable domain. See Chothia C and Lesk AM, 1987, J Mol Biol, 196:901-917; Chothia C et al, 1989, Nature, 342:878-883. "Framework" residues or "FR" residues are those variable domain residues other than the hypervariable region residues as defined herein. In certain embodiments, the CDRs contained in the antibody or antigen-binding fragment of the invention are preferably determined by the Kabat or IMGT numbering systems. One skilled in the art can definitively assign each numbering system to any variable domain sequence without reliance on any experimental data beyond the sequence itself. For example, Kabat residue numbering for a given antibody can be determined by aligning the antibody sequence with "standard" numbering sequences for homologous regions. Determining the numbering for any variable region sequence in the sequence listing based on the numbering schemes provided herein is entirely within the routine skill of those in the art.

The term "isolated," as used herein with reference to nucleic acids (such as DNA or RNA), refers to molecules separated from other DNA or RNA molecules that exist as macromolecules in a natural source. The term "isolated," as used herein, also refers to nucleic acids or polypeptides that are substantially free of cellular material, viral material, or culture medium when produced by recombinant DNA technology, or substantially free of chemical precursors or other chemicals when prepared by chemical synthesis. Furthermore, an "isolated nucleic acid" refers to a nucleic acid fragment that is not naturally occurring and is not found in its natural state. The term "isolated" as used herein also refers to cells or polypeptides separated from other cellular proteins or tissues. An isolated polypeptide includes purified and recombinant polypeptides.

The term "cross-reactivity" refers to the ability of an antibody as described herein to bind antigens from different species. Cross-reactivity can be measured by detecting specific reactivity with purified antigen in a binding assay (e.g., SPR, ELISA), binding to cells physiologically expressing the antigen, or otherwise functionally interacting with cells physiologically expressing the antigen. Examples of assays known in the art for determining binding affinity include surface plasmon resonance (e.g., Biacore) or similar techniques (e.g., Kinexa or Octet).

The terms "immunobinding" and "immunobinding properties" refer to non-covalent interactions that occur between an immunoglobulin molecule and an antigen for which the immunoglobulin is specific. The strength or affinity of an immunobinding interaction can be expressed as the equilibrium dissociation constant (KD) of the interaction, where a smaller KD value indicates higher affinity. The immunobinding properties of a selected polypeptide can be determined using methods well known in the art. One assay method involves measuring the rates of antigen/antibody complex formation and dissociation. Both the "association rate constant" (ka or kon) and the "dissociation rate constant" (kd or koff) can be calculated from concentrations and the actual rates of association and dissociation (see Malmqvist M, 1993, Nature, 361:186-187). The ratio ka/kd equals the equilibrium dissociation constant KD (see Davies DR et al, 1990, Annual Rev Biochem, 59:439-473). KD, ka, and kd values can be measured using any effective method.

The term "host cell" refers to a cell in which a vector can be propagated and its DNA can be expressed, the cell may be a prokaryotic or eukaryotic cell. The term also includes any progeny of the subject host cell. It should be understood that not all progeny is identical to the parental cell, as mutations may occur during replication; such progeny are included. Host cells include prokaryotic cells, yeast, or mammalian cells, such as CHO cells, NS0 cells, or other mammalian cells.

The term "identity" is used to refer to the match between two polypeptides or two nucleic acids. When a position in both compared sequences is occupied by the same base or amino acid monomer subunit (e.g., adenine occupies a certain position in each of two DNA molecules, or lysine occupies a certain position in each of two polypeptides), then the molecules are identical at that position. The "percent identity" between two sequences is a function of the number of matching positions shared by the two sequences divided by the number of positions compared × 100. For example, if 6 out of 10 positions in two sequences match, the two sequences have 60% identity. For example, the DNA sequences CTGACT and CAGGTT share 50% identity (3 matching positions out of a total of 6). Typically, the comparison is made when the two sequences are aligned to give maximum identity. Such alignments can be conveniently performed using computer programs such as the Align program (DNAstar, Inc.), using the method of Needleman and Wunsch (Needleman SB and Wunsch CD, 1970, J Mol Biol, 48:443-453).

The terms "mutated," "mutant," and "mutation" refer respectively to the substitution, deletion, or insertion of one or more nucleotides or amino acids compared to a native nucleic acid or polypeptide (i.e., a reference sequence that can be used to define the wild-type).

The term "conservative modification" is intended to refer to amino acid modifications that do not significantly affect or alter the binding characteristics of an antibody containing the amino acid sequence. Such conservative modifications include amino acid substitutions, additions, and deletions. Modifications can be introduced into the antibodies of the invention using standard techniques known in the art, such as site-directed mutagenesis and PCR-mediated mutagenesis. Conservative amino acid substitution refers to the replacement of an amino acid residue with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine), and aromatic side chains (e.g., tyrosine, phenylalanine, tryptophan, histidine). Therefore, one or more amino acid residues in the CDR region of an antibody of the invention can be replaced with other amino acid residues from the same side chain family.

The antibody of the present invention, or the nucleic acid or polynucleotide encoding the antibody of the present application, can be used in the preparation of pharmaceutical compositions or sterile formulations. For example, the antibody may be mixed with a pharmaceutically acceptable carrier, excipient, or stabilizer. The pharmaceutical composition may include one or a combination (e.g., two or more different) of the antibodies of the present invention. For instance, a pharmaceutical composition of the present invention may comprise a combination of antibodies or antibody fragments (or immunoconjugates) with complementary activities binding to different epitopes on the target antigen. Formulations of therapeutic and diagnostic agents can be prepared by mixing with pharmaceutically acceptable carriers, excipients, or stabilizers in forms such as lyophilized powders, slurries, aqueous solutions, or suspensions. The term "pharmaceutically acceptable" means that when the molecule itself, fragment, or composition is appropriately administered to an animal or human, it does not produce adverse, allergic, or other undesirable reactions. Specific examples of substances that can serve as pharmaceutically acceptable carriers or components thereof include carbohydrates (e.g., lactose), starches, cellulose and its derivatives, vegetable oils, gelatin, polyols (e.g., propylene glycol), alginates, and the like. The antibody of the present invention, or the nucleic acid or polynucleotide encoding the antibody of the present application, may be used alone or in combination with one or more other therapeutic agents, such as vaccines.

The term "pharmaceutically acceptable carrier and/or excipient and/or stabilizer" refers to carriers and/or excipients and/or stabilizers that are pharmacologically and/or physiologically compatible with the subject and the active ingredient, and are non-toxic to cells or mammals exposed to them at the dosages and concentrations employed. This includes, but is not limited to: pH adjusters, surfactants, adjuvants, ionic strength enhancers, diluents, agents for maintaining osmotic pressure, agents delaying absorption, preservatives. For example, pH adjusters include but are not limited to phosphate buffers. Surfactants include but are not limited to cationic, anionic, or nonionic surfactants, such as Tween-80. Ionic strength enhancers include but are not limited to sodium chloride. Preservatives include but are not limited to various antibacterial and antifungal agents, such as parabens, chlorobutanol, phenol, sorbic acid, etc. Agents for maintaining osmotic pressure include but are not limited to sugars, NaCl and analogs thereof. Agents delaying absorption include but are not limited to monostearates and gelatin. Diluents include but are not limited to water, aqueous buffers (such as buffered saline), alcohols and polyols (such as glycerol), etc. Stabilizers have the meaning commonly understood by those skilled in the art, being able to stabilize the desired activity of the active ingredient in the drug, including but not limited to monosodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein) or degradation products thereof (such as lactalbumin hydrolysate), etc.

As used herein, the term "effective amount" refers to an amount sufficient to obtain or at least partially obtain the desired effect. For example, a prophylactically effective amount refers to an amount sufficient to prevent, inhibit, or delay the onset of a disease; a therapeutically effective amount refers to an amount sufficient to cure or at least partially arrest the disease and its complications in a patient already suffering from the disease. Determining such effective amounts is entirely within the capability of those skilled in the art. For example, an amount effective for therapeutic use will depend on the severity of the disease to be treated, the overall state of the patient's own immune system, the patient's general condition such as age, weight and sex, the mode of administration of the drug, and other concurrent therapies, etc.

The embodiments of the invention are further illustrated by the following examples, but those skilled in the art should understand that the following drawings and examples are only for illustrating the invention and are not intended to further limit the invention.

### Brief Description of the Drawings

Figure 1: ELISA of antibodies AB8D, Novo Nordisk's antibody Concizumab, and Pfizer's antibody Marstacimab binding to human TFPI.
Figure 2-1: Competition of antibodies AB8D, Novo Nordisk's antibody Concizumab, and Pfizer's antibody Marstacimab with AB8D-HRP for binding to human TFPI.
Figure 2-2: Competition of antibodies AB8D, Novo Nordisk's antibody Concizumab, and Pfizer's antibody Marstacimab with Concizumab-HRP for binding to human TFPI.
Figure 3: Schematic diagram of antibodies AB8D, Novo Nordisk's antibody Concizumab, and Pfizer's antibody Marstacimab binding to the TFPI K2 domain structure. The AB8D/TFPI K2 and Marstacimab/TFPI K2 complex structures were obtained via AlphaFold modeling prediction. The Concizumab/TFPI K2 structure is from the Protein Data Bank, PDB 4DTG.
Figure 4: Schematic diagram of superimposed modeled structures of antibodies AB8D, Novo Nordisk's antibody Concizumab, and Pfizer's antibody Marstacimab binding to the TFPI K2 domain. The three structures are superimposed via the common TFPI K2 domain.
Figure 5: Schematic diagram of superimposed modeled structures of antibody AB8D and coagulation factor FXa binding to the TFPI K2 domain. The AB8D/TFPI K2 and FXa/TFPI K2 complex structures are superimposed via the common TFPI K2 domain. TFPI K2 domain residues R107, Y109, and R124 are shown in space-filling models.
Figure 6: Schematic diagram of superimposed modeled structures of antibody Concizumab and coagulation factor FXa binding to the TFPI K2 domain. The Concizumab/TFPI K2 and FXa/TFPI K2 complex structures are superimposed via the common TFPI K2 domain. TFPI K2 domain residues R107 and Y109 are shown in space-filling models.
Figure 7: Schematic diagram of superimposed modeled structures of antibody Marstacimab and coagulation factor FXa binding to the TFPI K2 domain. The Marstacimab/TFPI K2 and FXa/TFPI K2 complex structures are superimposed via the common TFPI K2 domain. TFPI K2 domain residues R107, Y109, and L131 are shown in space-filling models.
Figure 8: Schematic diagram of antibody AB8D binding to the TFPI K2 domain. Antibody heavy chain residues E50 and H35, and TFPI K2 domain residue R124 are shown in space-filling models.

### Detailed Description

The invention is now described with reference to the following examples which are intended to illustrate, not limit, the invention.

Unless specifically indicated, the molecular biology experimental methods and immunoassays used in the present invention are performed substantially following the methods described in J. Sambrook et al., Molecular Cloning: A Laboratory Manual, 2nd edition, Cold Spring Harbor Laboratory Press, 1989, and F. M. Ausubel et al., Current Protocols in Molecular Biology, 3rd edition, John Wiley & Sons, Inc., 1995; the use of restriction enzymes follows the conditions recommended by the product manufacturers. Those skilled in the art understand that the examples describe the invention by way of illustration and are not intended to limit the scope of the invention as claimed.

### Example 1: Preparation of Anti-Human TFPI Murine Monoclonal Antibodies

Human TFPI antigen (protein sequence: NCBI Sequence No. NP_006278.1) at 50 µg per mouse was emulsified with complete Freund's adjuvant and used to immunize male Balb/C mice via multiple-point injection, with immunization cycles every three weeks. Ten days after the third immunization, blood was collected from the tail vein, and the anti-human TFPI antibody titer in plasma was tested by ELISA to monitor the immune response in the mice. Three days before fusion, the mouse with the highest anti-human TFPI antibody titer was given a booster immunization. Three days later, the mouse was euthanized, and its spleen was removed and fused with the mouse myeloma Sp2/0 cell line. A mixture of 2×10^8 Sp2/0 cells and 2×10^8 spleen cells was fused in a solution containing 50% polyethylene glycol (molecular weight 1450) and 5% dimethyl sulfoxide (DMSO). The spleen cell number was adjusted to 5×10^5/mL using Iscove's medium (containing 10% fetal bovine serum, 100 U/mL penicillin, 100 µg/mL streptomycin, 0.1 mM hypoxanthine, 0.4 µM aminopterin, and 16 µg thymidine), and 0.3 mL was added to each well of 96-well culture plates, which were then placed in a 37°C, 5% CO₂ incubator. After 10 days of culture, high-throughput ELISA was used to detect clones producing antibodies with high-affinity binding to TFPI in the supernatant. The fusion cells from the wells containing the above monoclonal antibodies were then subcloned, and further screening yielded hybridoma cell line #3-71.

Clones producing specific antibodies were cultured in RPMI 1640 medium supplemented with 10% FCS. When cell density reached approximately 5×10^5 cells/mL, the medium was replaced with serum-free medium. After 2 to 4 days, the conditioned medium was centrifuged to collect the culture supernatant. A Protein G column was used to purify the antibody. The monoclonal antibody eluate was dialyzed against 150 mM NaCl. The dialyzed solution was filter-sterilized through a 0.22 µm filter to obtain purified anti-human TFPI murine monoclonal antibody mAb3-71 for testing.

### Example 2: Affinity Determination and Kinetic Study of TFPI Murine Antibodies

Bio-Layer Interferometry (BLI) was used to determine the binding affinity constants of the purified anti-human TFPI murine monoclonal antibody mAb3-71 to human TFPI, using the ForteBio Octet RED&QK system from PALL Corporation. For parallel quantitative analysis across multiple channels, a concentration gradient was set at: 3.125, 6.25, 12.5, 25, 50, and 100 nM. His-tagged human TFPI at 10 µg/mL was immobilized on Ni-NTA sensors. The affinity determination results are shown in Table 1. The results show that the murine monoclonal antibody has extremely high binding affinity for human TFPI, with K_{D} values reaching the 10⁻¹² M range.

**Table 1. Affinity Determination Results for the Murine Monoclonal Antibody**

| **Antibody** | **K_{D} (M)** | **kon (1/Ms)** | **koff (1/s)** |
|---|---|---|---|
| mAb3-71 | 4.539E-12 | 2.893E+05 | 1.313E-06 |

### Example 3: Humanization of Anti-Human TFPI Murine Antibodies

The murine antibody was humanized using the CDR grafting method. The fundamental principle of CDR grafting involves transplanting the CDR regions of the murine antibody onto a human antibody template, while also introducing several or some key murine FR residues that are crucial for stabilizing the CDR conformation and for antigen-antibody binding into the human antibody template (backmutations). This achieves the dual goals of reducing the immunogenicity of the murine antibody while maintaining its affinity. In addition to the aforementioned CDR grafting operation, we further performed calculations on four aspects of the humanized antibody post-CDR grafting: isoelectric point (pI), hydrophobic aggregation, post-translational modifications (PTM, such as glycosylation, fragmentation, isomerization sites, etc.), and immunogenicity. Amino acids contributing to issues in these four aspects were mutated to enable the humanized antibody to fully exert its therapeutic efficacy in clinical use.

The specific workflow for antibody humanization is as follows. The human antibody germline database was searched to obtain human antibody templates with high similarity to the murine antibody. The CDR regions of the murine antibody and the human antibody templates were annotated, defining the CDR regions according to the Kabat or IMGT schemes. The six CDR regions of the murine antibody were used to replace the six CDR regions of the human antibody template. Each of the transplanted six CDR regions could be an amino acid region defined by Kabat or by IMGT. Following CDR grafting, backmutations from the murine antibody to the FR regions of the humanized template were performed. The key murine FR amino acids that stabilize the antibody CDR conformation and are important for antigen-antibody binding include four categories of amino acid residues: 1) Amino acids within 6 Å of the CDR regions that are buried beneath the antibody surface; 2) Amino acids within 6 Å of the CDR regions that are exposed on the antibody surface; 3) Interface amino acids between the antibody light and heavy chain domains; and 4) Vernier zone residues that stabilize the antibody CDR conformation (Foote J and Winter G, 1992, J Mol Biol, 224:487-499). These four categories of key murine FR residues were identified by constructing a three-dimensional structural model of the murine antibody. For amino acids in these four categories of the human template that differed from the murine antibody sequence, structural analysis was performed to select residues important for maintaining CDR conformation and antigen-antibody binding for transplantation or replacement from the murine antibody to the human template. Subsequently, the humanized antibody sequence resulting from the transplantation of these four categories of amino acids was further analyzed by calculating its isoelectric point, hydrophobic aggregation propensity, post-translational modifications, and immunogenicity. Problematic amino acids were mutated to obtain the final humanized antibody sequence.

Following the above methods, humanized antibodies were constructed based on the CDRs of the murine antibody mAb3-71. The amino acid sequences of the CDR regions contained in the variable regions of the relevant antibodies are shown in Table 2, and the amino acid sequences of the heavy and light chain variable regions are shown in Table 3

**Table 2. CDR Region Sequences of Exemplary Anti-TFPI Humanized Antibodies**

| **Antibody Number** | **CDR** | **Kabat** | **IMGT** |
|---|---|---|---|
| AB8D | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-1 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSHVDVAMDY (SEQ ID NO: 56) | ARSSHVDVAMDY (SEQ ID NO: 57) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-2 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVIDVAMDY (SEQ ID NO: 58) | ARSSVIDVAMDY (SEQ ID NO: 59) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-3 | HCDR1 | SYWMH (SEQ ID NO: 46) | GYTFTSYW (SEQ ID NO: 52) |
| | HCDR2 | EIKPDQGRIHYNEKFKT (SEQ ID NO: 60) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-4 | HCDR1 | SYWMH (SEQ ID NO: 46) | GYTFTSYW (SEQ ID NO: 52) |
| | HCDR2 | EIKPDHGRINYNEKFKT (SEQ ID NO: 61) | IKPDHGRI (SEQ ID NO: 62) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-5 | HCDR1 | SYWMH (SEQ ID NO: 46) | GYTFTSYW (SEQ ID NO: 52) |
| | HCDR2 | EIKPDKGRINYNEKFKT (SEQ ID NO: 63) | IKPDKGRI (SEQ ID NO: 64) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-6 | HCDR1 | SYWMH (SEQ ID NO: 46) | GYTFTSYW (SEQ ID NO: 52) |
| | HCDR2 | EIKPDQGRIDYNEKFKT (SEQ ID NO: 65) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-7 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSYYPFT (SEQ ID NO: 66) | HQWSYYPFT (SEQ ID NO: 66) |
| Antibody 1-8 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSFYPFT (SEQ ID NO: 67) | HQWSFYPFT (SEQ ID NO: 67) |
| Antibody 1-9 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWTHYPFT (SEQ ID NO: 68) | HQWTHYPFT (SEQ ID NO: 68) |
| Antibody 1-10 | HCDR1 | SYWMH (SEQ ID NO: 46) | GYTFTSYW (SEQ ID NO: 52) |
| | HCDR2 | EIKPDKGRINYNEKFKT (SEQ ID NO: 63) | IKPDKGRI (SEQ ID NO: 64) |
| | HCDR3 | SSVIDVAMDY (SEQ ID NO: 58) | ARSSVIDVAMDY (SEQ ID NO: 59) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-1 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDDAMDY (SEQ ID NO: 69) | ARSSVVDDAMDY (SEQ ID NO: 70) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-2 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDTAMDY (SEQ ID NO: 71) | ARSSVVDTAMDY (SEQ ID NO: 72) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-3 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDEAMDY (SEQ ID NO: 73) | ARSSVVDEAMDY (SEQ ID NO: 74) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-4 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVALDY (SEQ ID NO: 75) | ARSSVVDVALDY (SEQ ID NO: 76) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-5 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAIDY (SEQ ID NO: 77) | ARSSVVDVAIDY (SEQ ID NO: 78) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-6 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAEDY (SEQ ID NO: 79) | ARSSVVDVAEDY (SEQ ID NO: 80) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-7 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAVDY (SEQ ID NO: 81) | ARSSVVDVAVDY (SEQ ID NO: 82) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-8 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVADDY (SEQ ID NO: 83) | ARSSVVDVADDY (SEQ ID NO: 84) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-9 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVFDVAMDY (SEQ ID NO: 85) | ARSSVFDVAMDY (SEQ ID NO: 86) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-10 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVYDVAMDY (SEQ ID NO: 87) | ARSSVYDVAMDY (SEQ ID NO: 88) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-11 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVLDVAMDY (SEQ ID NO: 89) | ARSSVLDVAMDY (SEQ ID NO: 90) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |

**Table 3. Amino Acid Sequences of Humanized Antibody Variable Regions**

| | **VH amino acid sequence** | **VL amino acid sequence** |
|---|---|---|
| AB8D | EVQLVQSGAELKKPGASVKLSCKAS GYTFTSYWMHWVRQTPGQGLEWI GEIKPDQGRINYNEKFKTRATLTADK STSTAYMELSSLRSEDTAVYYCARSS VVDVAMDYWGQGTTVTVSS SEQ ID NO: 2 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 3 |
| Antibody 1-1 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS HVDVAMDYWGQGTTVTVSS SEQ ID NO: 4 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 5 |
| Antibody 1-2 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VIDVAMDYWGQGTTVTVSS SEQ ID NO: 6 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 7 |
| Antibody 1-3 | EVQLVQSGAELKKPGASVKLSCKAS GYTFTSYWMHWVRQAPGQGLEWI GEIKPDQGRIHYNEKFKTRATLTADK STSTAYMELSSLRSEDTAVYYCARSS VVDVAMDYWGQGTTVTVSS SEQ ID NO: 8 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 9 |
| Antibody 1-4 | EVQLVQSGAELKKPGASVKLSCKAS GYTFTSYWMHWVRQAPGQGLEWI GEIKPDHGRINYNEKFKTRATLTADK STSTAYMELSSLRSEDTAVYYCARSS VVDVAMDYWGQGTTVTVSS SEQ ID NO: 10 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 11 |
| Antibody 1-5 | EVQLVQSGAELKKPGASVKLSCKAS GYTFTSYWMHWVRQAPGQGLEWI GEIKPDKGRINYNEKFKTRATLTADK STSTAYMELSSLRSEDTAVYYCARSS VVDVAMDYWGQGTTVTVSS SEQ ID NO: 12 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 13 |
| Antibody 1-6 | EVQLVQSGAELKKPGASVKLSCKAS GYTFTSYWMHWVRQAPGQGLEWI GEIKPDQGRIDYNEKFKTRATLTADK STSTAYMELSSLRSEDTAVYYCARSS VVDVAMDYWGQGTTVTVSS SEQ ID NO: 14 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 15 |
| Antibody 1-7 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVAMDYWGQGTTVTVSS SEQ ID NO: 16 | EIVLTQSPATLSLSPGERATLSCSASS SVSSSYLYWYQQKPGQAPRLWISGT SILASGVPARFSGSGSGTKYTLTISSL EPEDFAVYFCHQWSYYPFTFGQGTK LEIK SEQ ID NO: 17 |
| Antibody 1-8 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVAMDYWGQGTTVTVSS SEQ ID NO: 18 | EIVLTQSPATLSLSPGERATLSCSASS SVSSSYLYWYQQKPGQAPRLWISGT SILASGVPARFSGSGSGTKYTLTISSL EPEDFAVYFCHQWSFYPFTFGQGTK LEIK SEQ ID NO: 19 |
| Antibody 1-9 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVAMDYWGQGTTVTVSS SEQ ID NO: 20 | EIVLTQSPATLSLSPGERATLSCSASS SVSSSYLYWYQQKPGQAPRLWISGT SILASGVPARFSGSGSGTKYTLTISSL EPEDFAVYFCHQWTHYPFTFGQGTK LEIK SEQ ID NO: 21 |
| Antibody 1-10 | EVQLVQSGAELKKPGASVKLSCKAS GYTFTSYWMHWVRQAPGQGLEWI GEIKPDKGRINYNEKFKTRATLTADK STSTAYMELSSLRSEDTAVYYCARSS VIDVAMDYWGQGTTVTVSS SEQ ID NO: 22 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 23 |
| Antibody 2-1 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDDAMDYWGQGTTVTVSS SEQ ID NO: 24 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 25 |
| Antibody 2-2 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDTAMDYWGQGTTVTVSS SEQ ID NO: 26 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 27 |
| Antibody 2-3 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDEAMDYWGQGTTVTVSS SEQ ID NO: 28 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 29 |
| Antibody 2-4 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVALDYWGQGTTVTVSS SEQ ID NO: 30 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 31 |
| Antibody 2-5 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVAIDYWGQGTTVTVSS SEQ ID NO: 32 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 33 |
| Antibody 2-6 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVAEDYWGQGTTVTVSS SEQ ID NO: 34 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 35 |
| Antibody 2-7 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVAVDYWGQGTTVTVSS SEQ ID NO: 36 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 37 |
| Antibody 2-8 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VVDVADDYWGQGTTVTVSS SEQ ID NO: 38 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 39 |
| Antibody 2-9 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VFDVAMDYWGQGTTVTVSS SEQ ID NO: 40 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 41 |
| Antibody 2-10 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VYDVAMDYWGQGTTVTVSS SEQ ID NO: 42 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 43 |
| Antibody 2-11 | EVQLVQSGAELKKPGASVKLSCKAS GYTFTSYWMHWVRQAPGQGLEWI GEIKPDQGRINYNEKFKTRATLTADK STSTAYMELSSLRSEDTAVYYCARSS VLDVAMDYWGQGTTVTVSS SEQ ID NO: 44 | EIVLTQSPATLSLSPGERATLSCSASS SVSSSYLYWYQQKPGQAPRLWISGT SILASGVPARFSGSGSGTKYTLTISSL EPEDFAVYFCHQWSHYPFTFGQGTK LEIK SEQ ID NO: 45 |

To obtain full-length antibody sequences composed of two heavy chains and two light chains, the VH and VL sequences shown in Table 3 can be spliced or assembled with antibody heavy chain constant region sequences (preferably selected from human IgG1, IgG2, or IgG4) and light chain constant region sequences (preferably from human kappa light chain) using conventional techniques. Preferably, the heavy chain constant region is a human wild-type heavy chain constant region or a mutant thereof. Preferably, the heavy chain constant region is the heavy chain constant region of human IgG4, and the heavy chain constant region contains the S228P amino acid mutation.

### Example 4: Construction, Expression, and Preparation of Anti-Human TFPI Antibody Expression Vectors

Based on the heavy and light chain sequences obtained in the above examples, cDNA encoding them was designed and inserted into the pcDNA3.1 eukaryotic expression vector to construct humanized expression vectors. This expression vector plasmid contains the cytomegalovirus early gene promoter-enhancer required for high-level expression in mammalian cells. Simultaneously, the vector plasmid contains a selectable marker gene conferring kanamycin resistance in bacteria and G418 resistance in mammalian cells. Additionally, the vector plasmid contains a dihydrofolate reductase (DHFR) gene, allowing for co-amplification of the antibody gene and the DHFR gene with methotrexate (MTX) in suitable host cells.

The constructed recombinant expression vector plasmids described above were transfected into a mammalian host cell line to express the humanized antibodies. For stable, high-level expression, the preferred host cell line is a DHFR-deficient Chinese Hamster Ovary (CHO) cell line (see U.S. Pat. No. 4,818,679). The preferred transfection method is electroporation, although other methods, including calcium phosphate co-precipitation, lipofection, and protoplast fusion, can also be used. For electroporation, using a GenePulser (Bio-Rad Laboratories) set to 300 V electric field and 1050 µF capacitance, 2×10⁷ cells suspended in 0.8 mL of PBS containing 20 µg of the expression vector plasmid were added to a cuvette. Two days after transfection, G418 (0.2 mg/mL) and 200 nM MTX (Sigma) were added to the medium. To achieve higher expression levels, the transfected antibody genes were co-amplified with the MTX-selectable DHFR gene. Transfectants were subcloned by limiting dilution, and the secretion rates of individual cell lines were determined by ELISA. Cell lines producing antibodies at high levels were selected. Conditioned medium containing the antibodies was collected for assessing their *in vitro* and *in vivo* biological activities.

### Example 5: Purification and Characterization of Anti-Human TFPI Antibodies

This example describes the purification and characterization methods for the humanized antibody AB8D. Cell culture supernatant underwent clarification steps such as high-speed low-temperature centrifugation or depth filtration, followed by 0.22 µm sterile filtration. Purification was then performed using a three-step chromatography process: Protein A affinity, anion exchange, and cation exchange, as detailed below. The first capture step used Protein A affinity chromatography, with PBS buffer as the equilibration buffer. Elution was performed using an elution buffer (50 mM NaAc-HAc, pH 3.7) in a linear gradient. The pH of the Protein A eluate was adjusted to 3.6-3.8 by adding 1M glacial acetic acid and incubated at room temperature for 60 minutes for virus inactivation. Then, 2M Tris was added to neutralize the pH to 5.5. For intermediate purification, the anion exchange resin Q Sepharose FF was selected to remove residual DNA, endotoxins, and impurity proteins, using 50 mM NaAc-HAc, pH 5.5, as the equilibration buffer. The protein flow-through from the anion exchange step was directly loaded onto the cation exchange resin Monomix HC45-SP. The cation exchange chromatography equilibration buffer was 50 mM NaAc-HAc, pH 5.5, and elution was performed using an elution buffer (50 mM NaAc-HAc, 0.2 M NaCl, pH 5.5) in a linear gradient. The protein solution eluted from the cation exchange resin was subjected to nanofiltration, ultrafiltration/diafiltration for buffer exchange and concentration, and sterile filtration to obtain the drug substance.

The drug substance was analyzed for purity by SEC-HPLC and by SDS-PAGE. The SEC-HPLC results showed that the main peak purity of the purified antibody exceeded 99%. The theoretical molecular weight of the AB8D antibody is approximately 145 kDa. Under reducing conditions, SDS-PAGE showed a light chain at approximately 25 kDa and a heavy chain at approximately 50 kDa, which aligns well with the theoretical values.

### Example 6: Indirect ELISA Binding Assay for Anti-Human TFPI Antibodies

To compare the specific binding activity of antibody AB8D, Novo Nordisk's antibody Concizumab, and Pfizer's antibody Marstacimab to human TFPI, antibodies AB8D, Concizumab, and Marstacimab were quantified using a unified standard (i.e., concentration determined by Onedrop OD280 using the IgG setting). Simultaneously, the EC₅₀ values for the binding of antibodies AB8D, Concizumab, and Marstacimab to human TFPI were compared.

Human TFPI/His (purchased from ACRO Biosystem) was diluted to 0.1 µg/ml in 1× PBS buffer and added to a 96-well ELISA plate at 100 µl per well, then incubated at 4°C for 16-20 hours. The PBS buffer was discarded from the 96-well plate, and 200 µl per well of PBST/1.5% BSA was added for blocking, incubating at room temperature for 1 hour. After washing the plate 3 times with PBST (pH 7.4, PBS containing 0.05% Tween 20) buffer, the test TFPI antibodies were added at 100 µl per well and incubated at room temperature for 1.0 hour. After washing the plate 3 times with PBST, 100 µl per well of a 1:5000 dilution of HRP-conjugated goat anti-human IgG secondary antibody (purchased from The Jackson Laboratory) was added and incubated at room temperature for 1 hour. After washing the plate 3 times with PBST, 100 µl per well of TMB substrate was added and incubated at room temperature for 5-10 minutes. The reaction was stopped by adding 50 µl per well of 0.2 M sulfuric acid. Absorbance was read at dual wavelengths of 450/620 nm using a microplate reader.

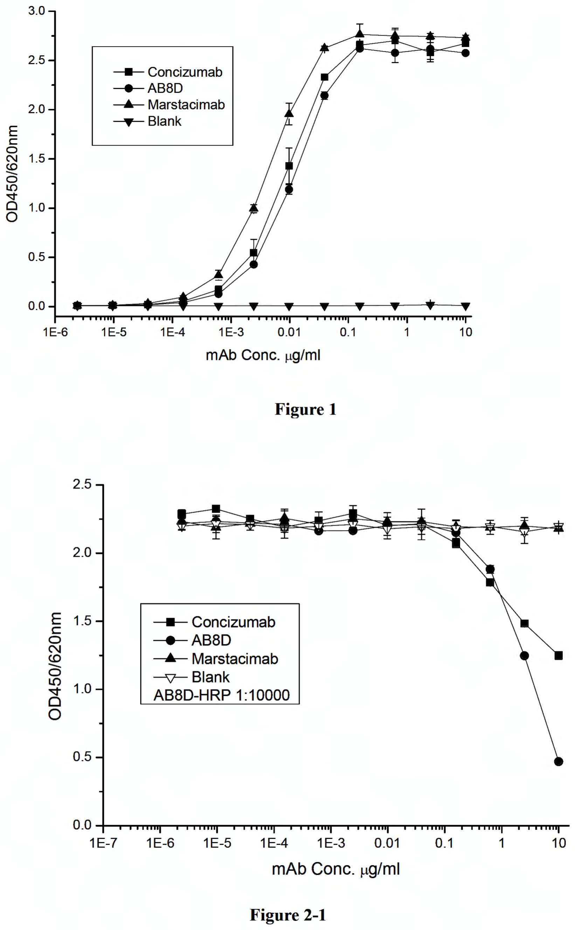

Figure 1 shows that antibody AB8D, Novo Nordisk's antibody Concizumab, and Pfizer's antibody Marstacimab all specifically bind to human TFPI. Pfizer's antibody Marstacimab showed stronger binding, while antibody AB8D and Novo Nordisk's antibody Concizumab exhibited similar binding strength. The EC₅₀ values for AB8D, Concizumab, and Marstacimab binding were: 9.28 ng/ml, 6.67 ng/ml, and 3.74 ng/ml, respectively.

### Example 7: Competition Binding Assay for Anti-Human TFPI Antibodies

To compare differences in the epitopes on human TFPI bound by antibody AB8D, Novo Nordisk's antibody Concizumab, and Pfizer's antibody Marstacimab, AB8D and Concizumab were labeled with HRP, respectively. Competition ELISAs were performed using antibodies AB8D, Concizumab, and Marstacimab competing with AB8D-HRP and Concizumab-HRP, respectively, to determine the differences in their binding epitopes on human TFPI.

Human TFPI/His (purchased from ACRO Biosystem) was diluted to 0.1 µg/ml in 1× PBS buffer and added to a 96-well ELISA plate at 100 µl per well, then incubated at 4°C for 16-20 hours. The PBS buffer was discarded from the 96-well plate, and 200 µl per well of PBST/1.5% BSA was added for blocking, incubating at room temperature for 1 hour. After washing the plate 3 times with PBST (pH 7.4, PBS containing 0.05% Tween 20) buffer, the test TFPI antibodies (starting at 10 µg/ml, pre-serially diluted in a U-bottom plate) were added simultaneously with 50 µl per well of a 1:10,000 dilution of either AB8D-HRP or Concizumab-HRP, and incubated at room temperature for 1.0 hour. After washing the plate 3 times with PBST, 100 µl per well of TMB substrate was added and incubated at room temperature for 5-10 minutes. The reaction was stopped by adding 50 µl per well of 0.2 M sulfuric acid. Absorbance was read at dual wavelengths of 450/620 nm using a microplate reader.

Figure 2-1 shows that Pfizer's antibody Marstacimab does not compete with AB8D-HRP, indicating they bind to different epitopes on human TFPI. Novo Nordisk's antibody Concizumab partially competes with AB8D-HRP, indicating their binding epitopes are similar and partially overlap but are not identical.

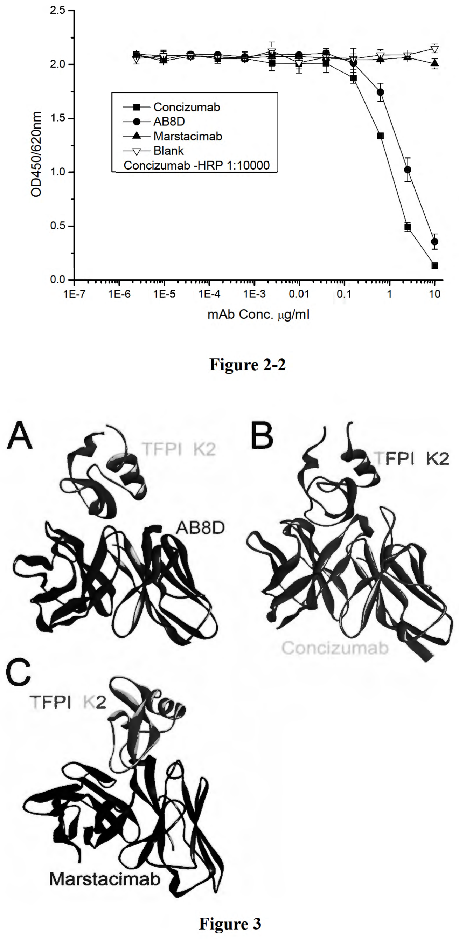

Figure 2-2 shows that Pfizer's antibody Marstacimab does not compete with Concizumab-HRP, indicating they bind to different epitopes on human TFPI. Antibody AB8D partially competes with Concizumab-HRP, indicating their binding epitopes are similar and partially overlap but are not identical.

### Example 8: pH-Dependent Indirect ELISA Binding Assay for Anti-Human TFPI Antibodies

### (1) Indirect ELISA Binding Assay

To compare the binding activity of antibody AB8D, Novo Nordisk's antibody Concizumab, and Pfizer's antibody Marstacimab to human TFPI under different pH conditions, antibodies AB8D, Concizumab, and Marstacimab were quantified using a unified standard (i.e., concentration determined by Onedrop OD280 using the IgG setting). Simultaneously, the EC₅₀ values for the binding of antibodies AB8D, Concizumab, and Marstacimab to human TFPI under different pH conditions were compared.

Human TFPI/His (purchased from ACRO Biosystem) was diluted to 0.1 µg/ml in 1× PBS buffer and added to a 96-well ELISA plate at 100 µl per well, then incubated at 4°C for 16-20 hours. The PBS buffer was discarded from the 96-well plate, and 200 µl per well of PBST/1.5% BSA was added for blocking, incubating at room temperature for 1 hour. After washing the plate 3 times with PBST (pH 7.4, PBS containing 0.05% Tween 20) buffer, the test TFPI antibodies (diluted in PBST/1.5% BSA buffer with pH values of 4.0, 4.5, 5.0, 5.5, 6.0, 6.5, and 7.4) were added at 100 µl per well and incubated at room temperature for 1.0 hour. After washing the plate 3 times with PBST, 100 µl per well of a 1:5000 dilution of HRP-conjugated goat anti-human IgG secondary antibody (purchased from The Jackson Laboratory) was added and incubated at room temperature for 1 hour. After washing the plate 3 times with PBST, 100 µl per well of TMB substrate was added and incubated at room temperature for 5-10 minutes. The reaction was stopped by adding 50 µl per well of 0.2 M sulfuric acid. Absorbance was read at dual wavelengths of 450/620 nm using a microplate reader.

Table 4-1 shows the EC₅₀ values for AB8D, Novo Nordisk's antibody Concizumab, and Pfizer's antibody Marstacimab binding to human TFPI under different pH conditions.

**Table 4-1. EC₅₀ Values for TFPI Antibodies Binding to Human TFPI under Different pH Conditions**

| | **Concizumab** | **AB8D** | **Marstacimab** |
|---|---|---|---|
| **pH** | **EC₅₀(ng/ml)** | **EC₅₀(ng/ml)** | **EC₅₀(ng/ml)** |
| 4.0 | 16.54 | 121.25 | 5.87 |
| 4.5 | 4.94 | 45.40 | 3.15 |
| 5.0 | 4.83 | 23.24 | 3.97 |
| 5.5 | 5.00 | 12.03 | 2.18 |
| 6.0 | 6.10 | 5.58 | 3.19 |
| 6.5 | 4.90 | 7.94 | 3.87 |
| 7.4 | 4.25 | 8.53 | 4.01 |

### (2) Affinity Determination Assay Using Bio-Layer Interferometry (BLI)

The affinity of the murine monoclonal antibody mAb3-71, AB8D, and Novo Nordisk's antibody Concizumab for human TFPI under pH 7.4 and pH 6.0 conditions was determined using the Sartorius Octet^{®} R8 biomolecular interaction analysis system based on Bio-layer Interferometry (BLI). This analysis aimed to assess the differences in the binding kinetics of these antibodies to human TFPI under physiological (pH 7.4) and acidic (pH 6.0) conditions.

Antibodies were captured as ligands using AMC2 probes (Sartorius, Cat. Nos. 18-5163). Recombinant human TFPI protein solutions at concentrations ranging from 100 nM to 6.25 nM were prepared in pH 7.4 PBST solution (1× PBS, 0.05% Tween 20) and used as analytes. The real-time binding of the antibodies to human TFPI, as well as their dissociation under either pH 7.4 or pH 6.0 conditions, was monitored. Affinity analysis was performed separately for each condition. All measurements were conducted at 30°C. BLI signals were acquired and saved using Octet BLI Discovery 12.2 software, and data processing was performed using Octet Analysis Studio 12.2 software. The signal from the reference channel (zero analyte concentration) was subtracted from the detection channel signals to obtain corrected binding curves. The kinetic curves were fitted according to the Langmuir 1:1 binding model to calculate the association rate constant (k<sub>on</sub>), dissociation rate constant (k<sub>off</sub>), and equilibrium dissociation constant (K<sub>D</sub>). The differences in dissociation rates between pH 7.4 and pH 6.0 conditions were compared to evaluate the pH dependency of the antibodies.

The association rate constant (kon), dissociation rate constant (koff), and equilibrium dissociation constant (K_{D}) values are presented in Table 4-2. The results show that the dissociation rate of the murine monoclonal antibody mAb3-71 at pH 6.0 is significantly lower compared to that at pH 7.4, indicating a pronounced pH dependency. The dissociation rate ratios for AB8D and Concizumab are 19.6 and 0.9, respectively, demonstrating that AB8D exhibits significant pH-dependent binding characteristics.

**Table 4-2. Comparison of the Affinity of Anti-TFPI Antibodies for Human TFPI at pH 7.4 and pH 6.0**

| **Sample** | **K_{D} (M)** | **kon (1/Ms)** | **koff (1/s)** | **Koff Ratio (pH6.0/pH7.4)** | **K_{D} Ratio (pH6.0/pH7.4)** |
|---|---|---|---|---|---|
| mAb3-71 pH7.4 | <1.0E-12 | 3.056E05 | <1.0E-07 | / | / |
| mAb3-71 pH6.0 | 2.422E-10 | 3.727E05 | 9.024E-05 | | |
| AB8D pH7.4 | 1.447E-12 | 4.472E05 | 6.473E-07 | 19.6 | 17.7 |
| AB8D pH6.0 | 2.565E-11 | 4.948E05 | 1.269E-05 | | |
| Concizumab pH7.4 | <1.0E-12 | 4.956E05 | 4.013E-07 | 0.9 | / |
| Concizumab pH6.0 | <1.0E-12 | 5.667E05 | 3.600E-07 | | |

Based on the real-time measurement of the binding of other humanized antibodies (Table 3) obtained in Example 3 to human TFPI and their dissociation under pH 7.4 or pH 6.0 conditions using the BLI technique described above, the results show that the other TFPI humanized antibodies listed in Table 3 also possess pH-dependent antigen-binding capabilities. Their K_{D} and/or koff ratios for TFPI at pH 6.0 / pH 7.4 are 2 or greater, preferably 10-fold or greater.

### Example 9: Determination of Antigenic Epitopes for Anti-Human TFPI Antibodies

The present invention utilized AlphaFold version 2.3.0 to predict the structure or the mode of interaction for a series of TFPI antigen/antibody complexes. The amino acid sequences used for AlphaFold modeling were the antibody light chain and heavy chain Fv region sequences along with the human TFPI protein K2 domain sequence (K93-L152). The specific Linux command is as follows: python3 docker/run_docker. py--fasta_ paths= multimer.fasta--max_template_date=2023-11-07--model_preset=multimer--data_dir=$DOWNL OAD_DIR-output_dir=/home/user/absolute_path_to_the_output_dir. In the above command line, run_docker.py is the Python executable application for AlphaFold version 2.3.0. multimer.fasta is the amino acid sequence file in FASTA format for the antigen/antibody. 2023-11-07 is the date; all structures in the Protein Data Bank prior to this date could serve as templates for modeling. multimer indicates that the molecule for this structure prediction is a multimer or complex molecule. $DOWNLOAD_DIR is the directory name on the disk where the search databases that come with AlphaFold itself are stored. absolute_path_to_the_output_dir should be filled in with the directory path for outputting or storing the prediction results.

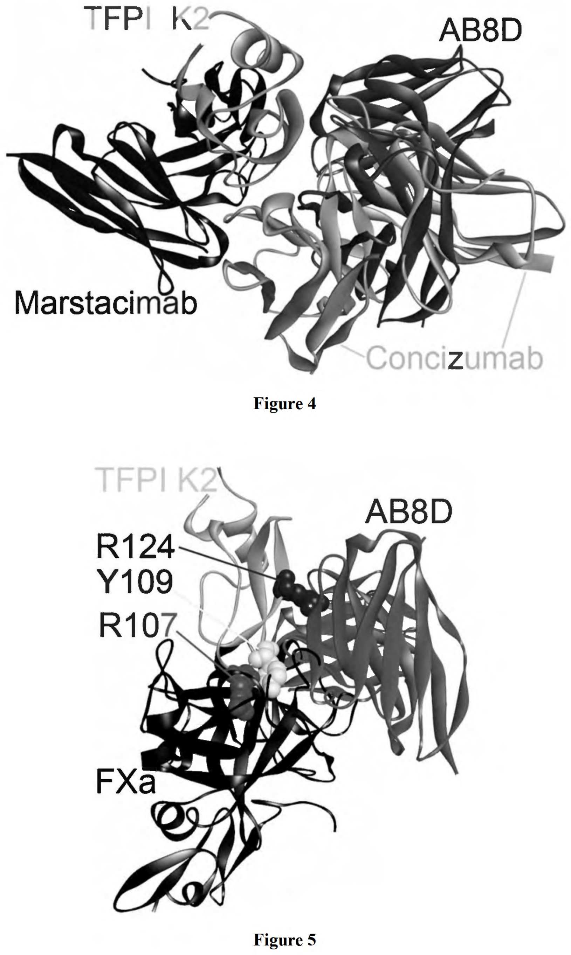

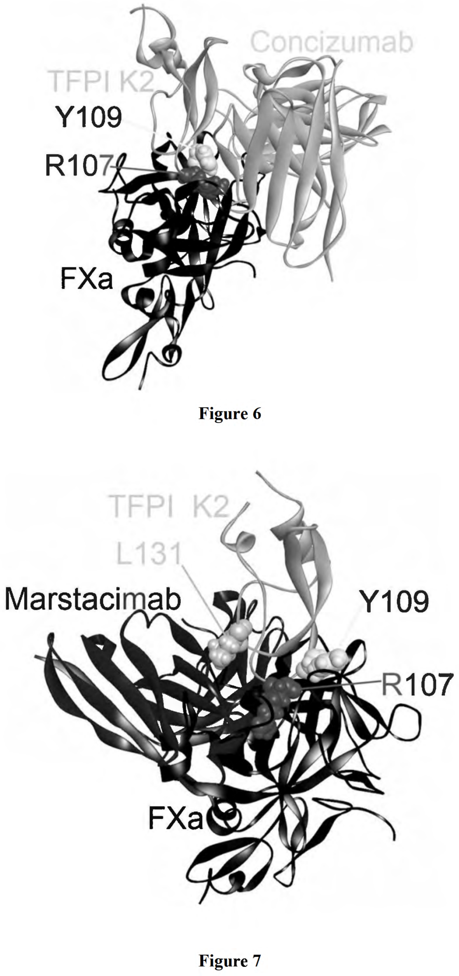

By default, AlphaFold uses 5 sets of modeling parameters, with 5 prediction seeds per parameter set, generating a total of 25 predicted structures. The predicted structures are scored for confidence, ranging from 0 to 100, with higher scores indicating greater confidence in the predicted structure. The AlphaFold modeling results for the antibody AB8D with TFPI K2 show that the confidence score for the AB8D/TFPI K2 complex is 86.00. The confidence score for the Marstacimab antibody with the TFPI K2 domain is 86.69. AlphaFold modeling for the TFPI K2 complex with coagulation factor FXa yielded a confidence score of 81.24. Schematic diagrams of the modeled structures for the antibody AB8D/TFPI K2, antibody Concizumab/TFPI K2, and antibody Marstacimab/TFPI K2 are shown in Figure 3. The structural superimposition of the three complexes-antibody AB8D/TFPI K2, antibody Concizumab/TFPI K2, and antibody Marstacimab/TFPI K2-is presented in Figure 4. The structural superimpositions of the antibody AB8D/TFPI K2 complex, antibody Concizumab/TFPI K2 complex, and antibody Marstacimab/TFPI K2 complex with the FXa/TFPI K2 complex are shown in Figures 5-7, respectively.

Based on the AlphaFold modeling results, antibody AB8D binds to the following amino acids within 5 Å on the TFPI K2 domain: T139, T119, E101, Y109, I110, T111, Y113, F114, N116, Q118, Q121, C122, E123, R124, F125, K126, E138, and L140 (Table 5). Pfizer's antibody Marstacimab binds to the following amino acids within 5 Å on the TFPI K2 domain: D102, G104-G108, I110, R112, Y127-M134, N136, E138. The crystal structure of Novo Nordisk's Concizumab antibody in complex with the TFPI K2 domain was obtained from the Protein Data Bank. Antibody Concizumab interacts with the following amino acids within 5 Å on the TFPI K2 domain: E100-P103, R107, Y109, T111, Y113-F114, N116, Q118, Q121-K126, N133, E138, L140. Coagulation factor FXa binds to the following TFPI amino acids within 5 Å on the TFPI K2 domain, including P103, I105-R112, R124, K126-L131, E138. TFPI K2 R107 is a central amino acid involved in inhibiting the active center of FXa. The shortest Cα-Cα atom distances between TFPI K2 R107 and antibodies AB8D, Concizumab, and Marstacimab are as follows. The shortest Cα-Cα distance between an amino acid residue of antibody AB8D and TFPI K2 amino acid residue R107 is 10.50 Å, between antibody AB8D light chain residue Y95 and TFPI K2 R107. The shortest Cα-Cα distance between an amino acid residue of antibody Concizumab and TFPI K2 amino acid residue R107 is 8.69 Å, between antibody Concizumab heavy chain residue Y59 and TFPI K2 R107. The shortest Cα-Cα distance between an amino acid residue of antibody Marstacimab and TFPI K2 amino acid residue R107 is 4.90 Å, between antibody Marstacimab heavy chain residue L104 and TFPI K2 R107. By comparing the antigenic epitope amino acids bound by the three antibodies (AB8D, Marstacimab, and Concizumab), it was found that two antigenic epitope amino acids, T139 and T119, which antibody AB8D binds and/or blocks within 5 Å, are not bound and/or blockd within 5 Å by the other two antibodies, Concizumab and Marstacimab. On the other hand, antibodies Concizumab and Marstacimab bind to TFPI K2 domain R107 within 5 Å. This amino acid directly participates in inhibiting coagulation factor FXa and is a central position in an active region. The antibody AB8D of the present invention does not directly bind R107 within 5 Å; however, antibody AB8D binds to the amino acid Y109, which is spatially near the R107 position, within a 5 Å distance. The Y109 amino acid in the TFPI K2 domain also has important interactions with coagulation factor FXa and is a significant amino acid for TFPI/FXa binding, secondary to R107. Due to the large binding head or CDR outer surface of the AB8D antibody, by binding Y109 and blocking the nearby R107 site, AB8D can block the binding interaction between TFPI and coagulation factor FXa. Superimposition of the AB8D/TFPI K2 and FXa/TFPI K2 complex structures, aligned via the common TFPI K2 domain, shows that the AB8D antibody has spatial blocking or steric hindrance with coagulation factor FXa, indicating that AB8D competes with FXa for binding to TFPI K2. Owing to the high affinity of the AB8D antibody, it thereby blocks the binding interaction between TFPI K2 and coagulation factor FXa.

**Table 5. Amino Acids Involved in AB8D/TFPI K2 Antibody/Antigen Interaction (within 5 Å)**

| **TFPI K2 amino acid** | **AB8D heavy chain amino acid** | **AB8D light chain amino acid** | **Interaction type** |
|---|---|---|---|
| E101 | R57 | / | Electrostatic attraction |
| Y109 | / | Y95 | H-bond |
| Y109 | / | H94 | Van der Waals force |
| Y109 | / | P96 | Van der Waals force |
| Y109 | E62 | / | Van der Waals force |
| I110 | / | H94 | Van der Waals force |
| T111 | / | W92 | H-bond |
| T111 | / | S93 | H-bond |
| Y113 | V102 | / | H-bond |
| Y113 | / | Y33 | Van der Waals force |
| Y113 | / | W92 | Van der Waals force |
| F114 | R57 | / | Van der Waals force |
| N116 | Q55 | / | H-bond |
| Q118 | Q55 | / | H-bond |
| T119 | D54 | / | Van der Waals force |
| Q121 | V101 | / | Van der Waals force |
| C122 | V101 | / | Van der Waals force |
| C122 | V102 | / | Van der Waals force |
| E123 | K52 | / | Electrostatic attraction |
| E123 | R57 | / | Electrostatic attraction |
| R124 | W33 | / | Cation-pi bond |
| R124 | E50 | / | Electrostatic attraction |
| R124 | S99 | / | H-bond |
| R124 | H35 | / | Van der Waals force |
| R124 | / | Y95 | H-bond |
| F125 | R57 | / | Van der Waals force |
| K126 | E62 | / | Electrostatic attraction |
| E138 | / | Y33 | H-bond |
| T139 | / | Y33 | Van der Waals force |
| L140 | V102 | / | Hydrophobic interaction |
| According to the AlphaFold modeling results, antibody AB8D binds to the following amino acids within 5 Å on the TFPI K2 domain: T139, T119, E101, Y109, I110, T111, Y113, F114, N116, Q118, Q121, C122, E123, R124, F125, K126, E138, and L140. | | | |

### Example 10: Antibody/Antigen Dynamics Simulation of Anti-Human TFPI Antibodies

Gromacs software version 2022.4 was used to perform a 500-nanosecond molecular dynamics simulation of the AB8D antibody/TFPI K2 complex. The simulation system was set up using the pdb2gmx tool within the Gromacs software package, employing the Charmm36 force field and the TIP3P water model. The initial antigen/antibody complex model was centered in a dodecahedral box, with the box edges positioned at least 10 Å away from the complex model. Water molecules were added to fill the remaining space in the dodecahedral box, dissolving the initial antigen/antibody complex model in the TIP3P water solvent. Subsequently, 0.15 M NaCl ions were added using the genion tool in Gromacs. After adding water molecules and 0.15 M NaCl to the simulation system, the system was energy-minimized by performing up to 50,000 steps of the steepest descent algorithm. Following energy minimization, the system was equilibrated through two position-restrained simulation phases-NVT (constant temperature and volume) and NPT (constant pressure and temperature)-each lasting 2 nanoseconds, to prepare the system for production dynamics simulation. Finally, a 500-nanosecond production dynamics simulation was conducted at 300 K (using the V-rescale thermostat) and 1 atmosphere (using the Parrinello-Rahman barostat). The Verlet cutoff scheme was applied for non-bonded interactions. Long-range electrostatic interactions were calculated using the Particle Mesh Ewald (PME) summation method. All bond lengths were constrained using the LINCS algorithm. The simulation time step was 2 femtoseconds, and low-precision coordinates of the simulation trajectory were saved every 0.5 nanoseconds. The complete 500-nanosecond simulation trajectory file contains 1,000 frames of the simulation system conformations.

The binding free energy calculation and per-residue binding free energy decomposition for a series of TFPI antigen/antibody complexes were performed using gmx_MMPBSA versions 1.6.0-1.6.1. The command for the gmx_MMPBSA binding free energy calculation is as follows: mpirun -np 40 gmx_MMPBSA -O -i mmpbsa.in -cs com.tpr -ci index.ndx -cg 1 13 -ct com_traj.xtc -cp topol.top -o FINAL_RESULTS_MMPBSA.dat -eo FINAL_RESULTS_MMPBSA.csv. In this command line, mpirun -np 40 initiates 40 simultaneous gmx_MMPBSA processes. mmpbsa.in is the input parameter file for the program. com.tpr is the topology parameter file for the antigen/antibody complex. index.ndx is the index file defining groups of atoms (e.g., residue ranges, NaCl ions, water molecules) in the simulation system. -cg 1 13 indicates that the binding free energy calculation is performed between group 1 and group 13 defined in index.ndx. com_traj.xtc is the simulation trajectory file with periodic boundary conditions removed. topol.top is the topology file used for the dynamics simulation. FINAL_RESULTS_MMPBSA.dat and FINAL_RESULTS_MMPBSA.csv are output data files in different formats. The command for gmx_MMPBSA per-residue binding free energy decomposition is similar to the one above: mpirun -np 40 gmx_MMPBSA -O -i mmpbsa.in -cs com.tpr -ct com_traj.xtc -ci index.ndx -cg 1 13 -cp topol.top -o FINAL_RESULTS_MMPBSA.dat -eo FINAL_RESULTS_MMPBSA.csv -do FINAL_DECOMP_MMPBSA.dat -deo FINAL_DECOMP_MMPBSA.csv. Here, FINAL_DECOMP_ MMPBSA.dat and FINAL_DECOMP_MMPBSA.csv are output files in different formats containing the decomposed binding free energy per amino acid. In the binding free energy calculation, the polar solvation energy of the solute is calculated using the Poisson-Boltzmann (PB) equation, whereas in the per-residue decomposition calculation, the polar solvation energy is calculated based on Generalized Born (GB) theory. Therefore, there may be slight differences between the total antigen/antibody binding free energy and the sum of the per-residue binding free energies.

The calculated antibody/antigen binding free energies for AB8D/TFPI K2, Concizumab/TFPI K2, and Marstacimab/TFPI K2 are presented in Table 6. The contributions of key antigenic amino acids to the antigen/antibody binding free energy from the per-residue decomposition calculation are shown in Table 7 (only amino acids with absolute free energy contributions greater than 0.5 kcal/mol are listed). In the AB8D/TFPI interaction, the TFPI R124 amino acid makes a significant contribution to the antigen/antibody binding free energy. The R124 amino acid exhibits strong electrostatic attraction with AB8D antibody heavy chain residues E50 and H35 (see Table 5), cation-π interaction with the indole ring of AB8D antibody heavy chain W33, hydrogen bonding with AB8D antibody heavy chain S99, and hydrogen bonding with AB8D antibody light chain Y95. It can be observed that the AB8D antibody has binding interactions with three amino acids in the TFPI K2 domain: Y113, L140, and Q121. The interaction forces between these three amino acids and either antibody Concizumab or Marstacimab are very weak, or in other words, these amino acids do not significantly contribute to the antigen/antibody binding free energy for Concizumab and Marstacimab. On the other hand, the AB8D antibody does not have significant interactions with the TFPI K2 R107 amino acid, whereas antibodies Concizumab and Marstacimab exhibit strong interactions with R107 (Table 7).

**Table 6. Antibody/Antigen Binding Free Energy**

| **Antibody/Antigen** | **Binding Free Energy (kcal/mol)** |
|---|---|
| AB8D/TFPI K2 | -54.86±15.2 |
| Concizumab//TFPI K2 | -60.98±15.67 |
| Marstacimab/TFPI K2 | -40.06±10.55 |

**Table 7. Contribution of Key TFPI Antigenic Amino Acids to Antigen/Antibody Binding Free Energy from Per-Residue Decomposition Calculation**

| **Serial Numbe r** | **Concizuma b/ TFPI K2 Amino Acid** | **Contributio n to Binding Free Energy (kcal/mol)** | **Marstacima b/ TFPI K2 Amino Acid** | **Contributio n to Binding Free Energy (kcal/mol)** | **AB8D/TF PI K2 Amino Acid** | **Contributio n to Binding Free Energy (kcal/mol)** |
|---|---|---|---|---|---|---|
| 1 | Y109 | -3.96±1.63 | L131 | -6.65±0.67 | R124 | -19.3±1.78 |
| 2 | R107 | -3.5±2.52 | I105 | -4.28±0.57 | Y109 | -3.18±1.03 |
| 3 | E100 | -2.33±2.88 | C130 | -4.00±0.44 | I110 | -3.11±0.82 |
| 4 | N116 | -2.05±1.3 | R107 | -3.52±1.85 | T111 | -2.58±2.04 |
| 5 | R124 | -1.86±1.32 | C106 | -2.74±0.38 | Y113 | -1.26±0.95 |
| 6 | Q118 | -1.59±1.09 | Y127 | -2.01±0.72 | L140 | -0.88±0.27 |
| 7 | T111 | -1.47±2.09 | M134 | -0.92±0.72 | Q121 | -0.6±0.28 |
| 8 | K126 | -0.9±2.98 | G108 | -0.62±0.2 | K126 | 0.51±1.52 |
| 9 | L99 | -0.64±0.49 | E138 | 1.48±0.59 | E101 | 0.57±1.17 |
| 10 | P103 | -0.6±0.29 | D102 | 1.76±0.42 | E123 | 0.96±1.07 |
| 11 | T119 | -0.54±0.28 | / | / | / | / |
| 12 | E101 | 0.66±1.14 | / | / | / | / |
| 13 | D102 | 0.91±0.5 | / | / | / | / |
| 14 | E123 | 1.01±0.49 | / | / | / | / |

### Example 11: Molecular Structural Basis for pH-Dependent Changes in Affinity of Anti-Human TFPI Antibodies for the TFPI K2 Antigen

Interacting salt bridges or ion pairs between amino acids exhibit very strong interaction forces. When the ion-paired amino acids are uncharged, relatively strong hydrogen bonding interactions may still exist, but they are significantly weaker than the interactions in salt bridges or ion pairs. Changes in antibody/antigen affinity with pH are primarily due to the presence of weakly ionizable amino acids at the antibody/antigen interface that participate in binding via salt bridges or ion pairs. When the pH varies near the pKa of such a weakly ionizable amino acid, it alters the protonation state of that amino acid, thereby changing the antibody/antigen affinity. Under pH < 7 conditions, weakly ionizable amino acids such as histidine, glutamic acid, and aspartic acid undergo significant protonation changes. The following discusses the predicted and described structural basis for the changes in affinity of antibodies AB8D, Concizumab, and Marstacimab for the TFPI K2 antigen at pH < 7.Tables 5, 8, and 9 list the interacting amino acids between the three antibodies (AB8D, Concizumab, Marstacimab) and the TFPI K2 antigen, respectively. As can be seen from Table 9, there is no significant electrostatic interaction in the form of salt bridges or ion pairs between antibody Marstacimab and antigen TFPI K2. Therefore, the affinity between Marstacimab and TFPI K2 should be largely unaffected by pH changes under pH < 7 conditions. In Tables 5 and 8, AB8D and Concizumab each have roughly five pairs of electrostatic interactions; pH changes may influence the strength of these attractive forces.

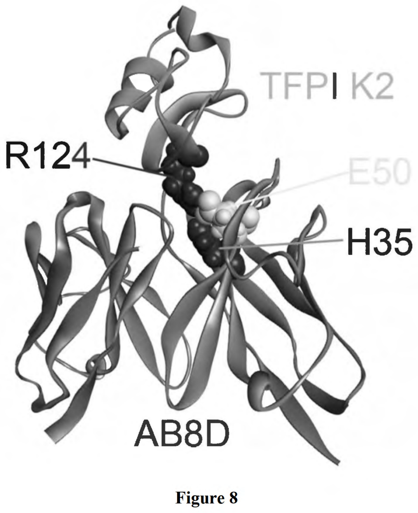

Tables 10 and 11 present the pKa values (calculated using the propka3 program) for the weakly ionizable Glu/Asp amino acids at the binding interfaces of the AB8D/TFPI K2 and Concizumab/TFPI K2 complexes, respectively. In Table 10, E50 on the AB8D heavy chain has a pKa much higher than the typical pKa of glutamic acid (4.50). Heavy chain amino acid E50 participates in the interaction with TFPI K2 amino acid R124 (Figure 8) and contributes significantly to the AB8D/TFPI K2 affinity (Tables 7, 12, and 13). Therefore, as the pH decreases from 7 to 6, given that the pKa of E50 is 6.75, the pH change will alter the protonation state of E50, reducing its degree of ionization, thereby decreasing the AB8D/TFPI K2 affinity, and this decrease is expected to be substantial (Table 15). Another notable feature of the AB8D antibody/antigen structure is that AB8D heavy chain histidine H35 is spatially adjacent to heavy chain E50 and participates in the interaction with TFPI K2 R124 (Figure 8), with its side chain located 3.51 Å from TFPI K2 R124. At pH 7.4, the interaction type between H35 and TFPI K2 R124 is van der Waals forces. When the pH decreases, e.g., from pH 7.4 to 4.0, histidine H35 may become protonated, leading to electrostatic repulsion with TFPI K2 R124. In summary, on one hand, the high pKa (6.75) of heavy chain E50 leads to a decrease in the E50/TFPI K2 R124 electrostatic attraction as pH lowers. On the other hand, the pH decrease may cause protonation of the heavy chain H35 side chain and subsequent H35/TFPI K2 R124 electrostatic repulsion. Therefore, the combined effects of E50 and H35 likely contribute to the observed pH-dependent antigen-binding behavior of the AB8D antibody. Furthermore, since the TFPI K2 R124 amino acid contributes substantially to the AB8D antigen/antibody binding free energy, AB8D heavy chain residues E50 and/or H35, which interact with TFPI K2 R124, are likely the primary amino acids responsible for the pH-dependent phenomenon observed for antibody AB8D in the pH range of 7.4-4.5.In Table 10, TFPI K2 E101 participates in the E101/heavy chain R57 interaction (see Tables 7 and 13). Both amino acids contribute significantly to the binding free energy. Therefore, when the pH changes from 5 to 4, crossing the pKa of E101 (4.50), the affinity between AB8D and TFPI K2 is expected to decrease substantially. Heavy chain E62 (pKa=3.71) participates in the electrostatic attraction with TFPI K2 K126. Both amino acids contribute little to the binding free energy (less than 0.5 kcal/mol or 0.51 kcal/mol, respectively; refer to Tables 7 and 13; the absolute contribution of E62 is <0.5 kcal/mol and thus not listed in Table 13). In Table 10, TFPI K2 E123 (pKa=3.36) participates in the electrostatic interaction with heavy chain K52, contributing 0.96 kcal/mol and -0.57 kcal/mol, respectively, which is not a major contribution. Consequently, when the pH drops below 3.71-3.36, the decrease in antibody/antigen affinity caused by heavy chain E62 or TFPI K2 E123 is not expected to be large.

Analyzing antibody Concizumab next, in Table 11, Concizumab heavy chain D106 (pKa=4.98) participates in an electrostatic interaction with TFPI K2 R124. These two amino acids have a certain degree of interaction force (refer to Tables 7, 8, and 14). Therefore, when the pH decreases to around 4.98, the affinity between Concizumab and TFPI K2 is expected to decrease. TFPI K2 E101 (pKa=4.96) participates in an electrostatic interaction with heavy chain R53. E101 contributes little to the binding free energy (0.66 kcal/mol), but R53 contributes significantly (-8.8 kcal/mol). Thus, when the pH drops to around 4.98, the affinity within this E101/R53 ion pair is expected to decrease substantially. E100 (pKa=4.68) also participates in an electrostatic interaction with heavy chain R53. E100 contributes significantly to the binding free energy (-2.33 kcal/mol, Table 7). Therefore, antibody affinity is expected to decrease substantially when the pH drops to around 4.68. In Table 11, light chain E31 (pKa=3.98) participates in an electrostatic interaction with TFPI K2 R124. Both amino acids contribute significantly to the binding free energy (Tables 7 and 14). When the pH drops to around 3.98, the binding force between Concizumab and TFPI K2 is expected to decrease considerably. Heavy chain D62 (pKa=3.93) participates in an electrostatic interaction with TFPI K2 R107. The contribution of heavy chain D62 to the binding free energy is less than 0.5 kcal/mol (not listed in Table 14), but TFPI K2 R107 contributes significantly (-3.5 kcal/mol, Table 7). Therefore, antibody affinity is still expected to decrease substantially when the pH drops to around 3.93. In Table 11, light chain D33 (pKa=1.79) is relatively far from the guanidinium group of TFPI K2 R124 (5.18 Å) and contributes little to the binding free energy (0.56 kcal/mol, Table 14). Consequently, when the pH drops to around 1.79, the change in antibody/antigen affinity caused by light chain D33 before and after protonation is not expected to be significant.

Based on the amino acid ionization formula, at pH = pKa, the ratio of protonated to deprotonated side chains is 1:1; when the pH is one unit below the pKa, the ratio becomes 10:1, at which point the amino acid side chain can be considered essentially uncharged. Therefore, summarizing the above, for antibody AB8D, when the pH drops to one unit below the relevant pKa values, i.e., pH 5.75 or 3.5, the antibody/antigen affinity is expected to decrease substantially in two stages. For antibody Concizumab, when the pH drops to approximately 4.0 or 3.0, the antibody/antigen affinity is expected to decrease substantially in stages.

Furthermore, a more precise description and prediction of the pH-dependent characteristics of antibody/antigen affinity could potentially be achieved by further calculating and comparing the antibody/antigen binding free energy for each weakly ionizable amino acid listed in Tables 10 and 11 in their protonated and deprotonated states (Table 15), and then discussing these changes in conjunction with the contributions of charged amino acids to the binding free energy from Tables 7, 13, and 14.

**Table 8. Amino Acids Involved in Concizumab/TFPI K2 Antibody/Antigen Interaction (within 5 Å)**

| **TFPI K2 Amino Acid** | **Concizumab heavy chain amino acid** | **Concizumab light chain amino acid** | **interaction type** |
|---|---|---|---|
| E100 | R53 | / | Electrostatic attraction, H-bond |
| E100 | S54 | / | H-bond |
| E100 | S52 | / | H-bond |
| E101 | R53 | / | Electrostatic attraction, H-bond |
| D102 | Y57 | / | Van der Waals force |
| P103 | Y57 | / | Hydrophobic interaction |
| P103 | Y59 | / | Hydrophobic interaction |
| R107 | D62 | / | Electrostatic attraction |
| R107 | F60 | / | H-bond |
| Y109 | / | F99 | Hydrophobic interaction |
| T111 | / | E31 | H-bond |
| Y113 | / | S32 | H-bond |
| Y113 | / | D33 | H-bond |
| F114 | R53 | / | Van der Waals force |
| N116 | R53 | / | H-bond |
| Q118 | R53 | / | H-bond |
| Q118 | N31 | / | Van der Waals force |
| Q121 | D103 | / | H-bond |
| C122 | Y102 | / | Van der Waals force |
| E123 | Y102 | / | Van der Waals force |
| R124 | D106 | / | Electrostatic attraction |
| R124 | / | E31 | Electrostatic attraction |
| R124 | / | Y37 | Cation-pi bond |
| R124 | / | A96 | H-bond |
| F125 | / | Y59 | Van der Waals force |
| K126 | / | E31 | Electrostatic attraction |
| K126 | / | T97 | H-bond |
| N133 | Y57 | / | Van der Waals force |
| E138 | / | S32 | Van der Waals force |
| L140 | / | D33 | Van der Waals force |
| L140 | Y102 | / | Van der Waals force |
| Antibody Concizumab binds to the following amino acids within 5 Å on the TFPI K2 domain: E100-P103, R107, Y109, T111, Y113-F114, N116, Q118, Q121-K126, N133, E138, L140. | | | |

**Table 9. Amino Acids Involved in Marstacimab/TFPI K2 Antibody/Antigen Interaction (within 5 Å)**

| **TFPI K2 amino acid** | **Marstacimab heavy chain amino acid** | **Marstacimab light chain amino acid** | **interaction type** |
|---|---|---|---|
| D102 | Y59 | / | H-bond |
| G104 | Y59 | / | Van der Waals force |
| I105 | Y59 | / | Hydrophobic interaction |
| I105 | A33 | / | Hydrophobic interaction |
| I105 | A50 | / | Hydrophobic interaction |
| C106 | L99 | / | Hydrophobic interaction |
| R107 | Y32 | / | Cation-pi bond |
| R107 | S31 | / | H-bond |
| R107 | G100 | / | H-bond |
| R107 | L104 | / | Van der Waals force |
| G108 | L104 | / | Van der Waals force |
| I110 | / | G32 | Van der Waals force |
| R112 | / | Y33 | Cation-pi bond |
| R112 | / | A31 | H-bond |
| Y127 | / | Y33 | Van der Waals force |
| G128 | L104 | | Van der Waals force |
| G129 | / | Y33 | Van der Waals force |
| C130 | / | Y93 | Hydrophobic interaction |
| C130 | / | Y33 | Van der Waals force |
| L131 | W47 | / | Hydrophobic interaction |
| L131 | Y59 | / | Hydrophobic interaction |
| L131 | A50 | / | Hydrophobic interaction |
| G132 | Y59 | / | Van der Waals force |
| G132 | / | S98 | Van der Waals force |
| N133 | / | S98 | Van der Waals force |
| M134 | / | S98 | Van der Waals force |
| M134 | / | S96 | Van der Waals force |
| N136 | / | Y33 | Van der Waals force |
| E138 | / | A31 | Van der Waals force |
| Pfizer's antibody Marstacimab binds to the following amino acids within 5 Å on the TFPI K2 domain: D102, G104-G108, I110, R112, Y127-M134, N136, E138. | | | |

**Table 10. pKa of Glu/Asp Amino Acids Participating in Interaction at the AB8D/TFPI K2 Antibody/Antigen Binding Interface**

| **Serial Number** | **AB8D heavy chain amino acid** | **AB8D light chain amino acid** | **TFPI K2 amino acid** | **pKa of antibody or TFPI monomer in the free/unbound state** |
|---|---|---|---|---|
| 1 | E50 | / | / | 6.75 |
| 2 | / | / | E101 | 4.50 |
| 3 | E62 | / | / | 3.71 |
| 4 | / | / | E123 | 3.36 |

**Table 11. pKa of Glu/Asp Amino Acids Involved in the Interaction at the Concizumab/TFPI K2 Antibody/Antigen Binding Interface**

| **Serial Number** | **Concizumab heavy chain amino acid** | **Concizumab light chain amino acid** | **TFPI K2 amino acid** | **pKa of antibody or TFPI monomer in the free/unbound state** |
|---|---|---|---|---|
| 1 | D106 | / | / | 4.98 |
| 2 | / | / | E101 | 4.96 |
| 3 | / | / | E100 | 4.68 |
| 4 | / | E31 | / | 3.98 |
| 5 | D62 | / | / | 3.93 |
| 6 | / | D33 | / | 1.79 |

**Table 12. Pairwise Decomposition of Binding Free Energy for Amino Acid Pairs Interacting with TFPI K2 Amino Acid R124 in the AB8D/TFPI K2 Interaction (pairwise energy decomposition)**

| **Serial Number (sorted by importance of antigen-antibody binding in descending order)** | **AB8D heavy chain amino acid paired with TFPI K2 R124** | **AB8D light chain amino acid paired with TFPI K2 R124** | **Contribution to binding free energy from interaction with R124 (kcal/mol))** |
|---|---|---|---|
| 1 | E50 | / | -22.51 |
| 2 | S100 | / | -5.38 |
| 3 | W33 | / | -3.93 |
| 4 | S99 | / | -3.43 |
| 5 | D103 | / | -3.08 |
| 6 | V101 | / | -3.00 |
| 7 | V104 | / | -2.82 |
| 8 | V102 | / | -1.23 |
| Intra-residue interaction energy of R124 | / | / | 26.33 |

**Table 13. Contribution of AB8D Antibody Amino Acids to the AB8D/TFPI K2 Interaction Free Energy**

| **Serial Number(sorted by importance of antigen-antibody binding in descending order)** | **AB8D heavy chain amino acid** | **AB8D light chain amino acid** | **Contribution to binding free energy (kcal/mol)** |
|---|---|---|---|
| 1 | E50 | / | -5.16 |
| 2 | R57 | / | -4.71 |
| 3 | / | Y95 | -4.48 |
| 4 | V102 | / | -3.49 |
| 5 | V101 | / | -3.29 |
| 6 | S100 | / | -2.56 |
| 7 | / | H94 | -1.95 |
| 8 | S99 | / | -1.69 |
| 9 | W33 | / | -1.62 |
| 10 | V104 | / | -1.44 |
| 11 | / | W92 | -1.44 |
| 12 | / | S93 | -0.89 |
| | / | W33 | -0.84 |
| 13 | N59 | / | -0.83 |
| 14 | D103 | / | -0.83 |
| 15 | K52 | / | -0.57 |
| 16 | K65 | / | 0.65 |
| 17 | H35 | / | 0.7 |
| 18 | D54 | / | 1.11 |

**Table 14. Contribution of Concizumab Antibody Amino Acids to the Concizumab/TFPI K2 Interaction Free Energy**

| **Serial Number (sorted by importance of antigen-antibody binding in descending order)** | **Concizumab heavy chain amino acid** | **Concizumab light chain amino acid** | **Contribution to binding free energy (kcal/mol)** |
|---|---|---|---|
| 1 | R53 | / | -8.8 |
| 2 | S54 | / | -4.38 |
| 3 | S52 | / | -4.1 |
| 4 | S56 | / | -3.59 |
| 5 | Y102 | / | -3.37 |
| 6 | Y57 | / | -3.03 |
| 7 | / | F99 | -2.52 |
| 8 | / | E31 | -1.61 |
| 9 | G55 | / | -1.33 |
| 10 | / | T97 | -0.79 |
| 11 | / | P100 | -0.66 |
| 12 | / | D33 | 0.56 |
| 13 | / | D1 | 0.92 |
| 14 | D106 | / | 1.13 |

**Table 15. AB8D/TFPI K2 Antibody/Antigen Binding Free Energy**

| **Antibody/Antigen** | **Binding Free Energy (kcal/mol)** |
|---|---|
| **AB8D/TFPI K2** (heavy chain E50 side chain negatively charged, heavy chain H35 side chain uncharged, under normal pH 7.4 conditions) | -54.86±15.2 |
| **AB8D/TFPI K2** (heavy chain E50 side chain uncharged, heavy chain H35 side chain uncharged) | -23.57 ±16.4 |
| **AB8D/TFPI K2** (heavy chain E50 side chain negatively charged, heavy chain H35 side chain positively charged) | -23.41±20.07 |

### Example 12: Calculation of pKa for Amino Acids at the Antigen/Antibody Binding Interface of Anti-Human TFPI Antibodies

Based on the content of the above examples, the pKa values for Glu and Asp amino acids, particularly heavy chain E50 and others, at the antigen/antibody binding interface were calculated for the other humanized antibodies obtained in Example 3. The results are presented in Tables 16-25. The results indicate that the humanized antibodies obtained in the present invention all exhibit similar pH-dependent binding characteristics.

**Table 16. pKa of Glu/Asp Amino Acids Involved in the Interaction at the Humanized Antibody 1-1/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.83 |
| 2 | / | / | E101 | 4.50 |
| 3 | E62 | / | / | 3.68 |
| 4 | / | / | E123 | 3.36 |

**Table 17. pKa of Glu/Asp Amino Acids Involved in the Interaction at the Humanized Antibody 1-2/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.75 |
| 2 | / | / | E101 | 4.50 |
| 3 | E62 | / | / | 3.71 |
| 4 | / | / | E123 | 3.36 |

**Table 18. pKa of Glu/Asp Amino Acids Involved in the Interaction at the Humanized Antibody 1-4/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.69 |
| 2 | / | / | E101 | 4.50 |
| 3 | E62 | / | / | 3.72 |
| 4 | / | / | E123 | 3.36 |

**Table 19. pKa of Glu/Asp Amino Acids Involved in the Interaction at the Humanized Antibody 1-5/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.72 |
| 2 | / | / | E101 | 4.50 |
| 3 | E62 | / | / | 3.70 |
| 4 | / | / | E123 | 3.36 |

**Table 20. pKa of Glu/Asp Amino Acids Involved in the Interaction at the Humanized Antibody 1-6/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 7.31 |
| 2 | / | / | E101 | 4.50 |
| 3 | E62 | / | / | 3.69 |
| 4 | / | / | E123 | 3.36 |

**Table 21. pKa of Glu/Asp Amino Acids Involved in the Interaction at the Humanized Antibody 1-7/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.40 |
| 2 | / | / | E101 | 4.50 |
| 3 | E62 | / | / | 3.74 |
| 4 | / | / | E123 | 3.36 |

**Table 22. pKa of Glu/Asp Amino Acids Involved in the Interaction at the Humanized Antibody 2-1/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 7.15 |
| 2 | / | / | E101 | 4.50 |
| 3 | E62 | / | / | 3.68 |
| 4 | / | / | E123 | 3.36 |

**Table 23. pKa of Glu/Asp Amino Acids Involved in the Interaction at the Humanized Antibody 2-4/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.25 |
| 2 | / | / | E101 | 4.50 |
| 3 | E62 | / | / | 3.69 |
| 4 | / | / | E123 | 3.36 |

**Table 24. pKa of Glu/Asp Amino Acids Involved in the Interaction at the Humanized Antibody 2-6/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 5.89 |
| 2 | / | / | E101 | 4.50 |
| 3 | E62 | / | / | 3.68 |
| 4 | / | / | E123 | 3.36 |

**Table 25. pKa of Glu/Asp Amino Acids Involved in the Interaction at the Humanized Antibody 2-9/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.40 |
| 2 | / | / | E101 | 4.50 |
| 3 | E62 | / | / | 3.71 |
| 4 | / | / | E123 | 3.36 |

### Example 13: Determination of Affinity for Anti-Human TFPI Antibodies

The binding affinity constants of the anti-human TFPI antibodies for human TFPI were determined according to the method described in Example 2. The affinity results are shown in Table 26. The results demonstrate that the anti-human TFPI monoclonal antibodies provided by the present invention exhibit extremely high binding affinity for human TFPI, with K_{D} values reaching the 10⁻¹² M range.

**Table 26. Affinity Determination Results for Anti-Human TFPI Antibodies**

| **Antibody** | **K_{D} (M)** |
|---|---|
| AB8D | 1.447E-12 |
| Antibody 1-1 | 1.124E-12 |
| Antibody 1-2 | 1.070E-12 |
| Antibody 1-3 | 2.457E-12 |
| Antibody 1-4 | 1.567E-12 |
| Antibody 1-5 | 2.231E-12 |
| Antibody 1-6 | <1.0E-12 |
| Antibody 1-7 | 1.326E-12 |
| Antibody 1-8 | 1.154E-12 |
| Antibody 1-9 | 1.578E-12 |
| Antibody 1-10 | 1.123E-12 |
| Antibody 2-1 | <1.0E-12 |
| Antibody 2-2 | 1.534E-12 |
| Antibody 2-3 | 1.321E-12 |
| Antibody 2-4 | 1.654E-12 |
| Antibody 2-5 | 1.263E-12 |
| Antibody 2-6 | 1.543E-12 |
| Antibody 2-7 | 1.492E-12 |
| Antibody 2-8 | 1.650E-12 |
| Antibody 2-9 | 1.325E-12 |
| Antibody 2-10 | 1.076E-12 |
| Antibody 2-11 | 1.651E-12 |

### Example 14: In Vitro Activity Study of Anti-Human TFPI Antibodies - TFPI Neutralization Assay: FVIIa/TF/FX Inhibition

TFPI is a crucial negative regulator of the extrinsic coagulation pathway. Its inhibitory mechanism involves the initial direct binding of the K2 domain to FXa, inhibiting FXa activity and forming a TFPI/FXa complex. Subsequently, the K1 domain of TFPI within the TFPI/FXa complex binds to FVIIa in the tissue factor complex (FVIIa/TF), forming a quaternary complex that inhibits FVIIa/TF activity and prevents further activation of FX to FXa. The TFPI monoclonal antibodies of the present invention are designed to bind to the K2 domain of TFPI, blocking its interaction with FXa and complex formation, thereby restoring the effect of the FVIIa/TF complex in activating FX and promoting FXa generation. In this assay, FVIIa, TF, TFPI, and FX were co-incubated with test substances. The absorbance value from the reaction of substrate S-2765 catalyzed by FXa was measured to evaluate the ability of the test substances to neutralize TFPI activity.

The research results demonstrate that all 22 humanized antibodies of the invention possess the biological activity of reversing TFPI-mediated inhibition of the FVIIa/TF/FX coagulation pathway and can neutralize TFPI activity. Within this assay system, the EC₅₀ values for the humanized antibodies of the invention ranged from 8.64 to 22.12 µg/mL.

### Example 15: In Vivo Pharmacodynamic Evaluation of Anti-Human TFPI Antibodies

Forty conventional-grade male New Zealand White rabbits were randomly divided into 5 groups based on body weight. Rabbits in each group received a single subcutaneous injection of the corresponding test substance or vehicle into the back of the neck on Day 1. All rabbits were induced to develop Hemophilia A (HA) by intravenous injection of an anti-FVIII antibody via the marginal ear vein on Day 8. During the experiment, all rabbits underwent nail clipping to induce stratum corneum bleeding 45 minutes after administration of the anti-FVIII antibody on Day 8. The initial bleeding time and blood loss volume were measured. Blood samples were collected at three time points: before HA model induction on Day 8 (before anti-FVIII antibody injection), before bleeding induction (45 minutes after anti-FVIII antibody injection on the same day), and 60 minutes after bleeding induction. Plasma samples were analyzed for Activated Partial Thromboplastin Time (APTT), Diluted Prothrombin Time (dPT), and Factor VIII (FVIII) activity.

The results showed that a single subcutaneous dose of 10 mg/kg of the positive control Marstacimab (a biosimilar of PF-06741086) significantly reduced blood loss and shortened the initial bleeding time to some extent 7 days post-administration. Single subcutaneous doses of AB8D at 5 mg/kg, 10 mg/kg, and 20 mg/kg resulted in a dose-dependent reduction in blood loss and shortening of the initial bleeding time 7 days post-administration (with both parameters showing statistically significant differences at the 20 mg/kg dose). A single subcutaneous dose of AB8D at 10 mg/kg and 20 mg/kg significantly shortened the dPT measured before and 105 minutes after model induction in HA rabbits 7 days post-administration. The results indicate that under the conditions of this study, AB8D can shorten the initial bleeding time and dPT, reduce blood loss, demonstrating a therapeutic effect.

**Table 27. Blood Loss and Initial Hemostasis Time (n=8)**

| **Group** | | **Bleeding Volume (g)** | **Initial Hemostasis Time (s)** |
|---|---|---|---|
| G1: Anti-FVIII antibody + Vehicle | Mean | 50.28 | 1817.63 |
| | SEM | 5.68 | 536.82 |
| G2: Anti-FVIII antibody + Marstacimab - 10 mg/kg | Mean | 10.75*** | 750.13 |
| | SEM | 1.31 | 416.81 |
| G3: Anti-FVIII antibody + AB8D - 5 mg/kg | Mean | 45.75 | 897.25 |
| | SEM | 3.42 | 397.43 |
| G4: Anti-FVIII antibody + AB8D - 10 mg/kg | Mean | 42.19 | 594.00 |
| | SEM | 4.03 | 115.75 |
| G5: Anti-FVIII antibody + AB8D - 20 mg/kg | Mean | 10.44*** | 415.25* |
| | SEM | 1.11 | 91.46 |

| | | | |
|---|---|---|---|
| Note: Compared with Group G1 at the same time point, *P<0.05; **P<0.01; ***P<0.001. The same applies below. | | | |

**Table 28. Diluted Prothrombin Time (n=7-8)**

| **Group** | | **dPT(s)** | | |
|---|---|---|---|---|
| | | **Day 8 0min** | **Day 8 45min** | **Day 8 105min** |
| G1: Anti-FVIII antibody + Vehicle | Mean | 130.26 | 125.58 | 128.36 |
| | SEM | 6.05 | 9.41 | 5.35 |
| G2: Anti-FVIII antibody + Marstacimab - 10 mg/kg | Mean | 113.53 | 112.91 | 121.16 |
| | SEM | 4.08 | 4.62 | 5.63 |
| G3: Anti-FVIII antibody + AB8D - 5 mg/kg | Mean | 122.91 | 124.32 | 124.80 |
| | SEM | 5.24 | 5.14 | 11.07 |
| G4: Anti-FVIII antibody + AB8D - 10 mg/kg | Mean | 102.13** | 104.19 | 102.92** |
| | SEM | 4.77 | 1.73 | 2.46 |
| G5: Anti-FVIII antibody + AB8D - 20 mg/kg | Mean | 104.68* | 104.07 | 100.51** |
| | SEM | 5.04 | 4.87 | 3.22 |

### Example 16: Safety Evaluation of Anti-Human TFPI Antibodies in Rhesus Monkeys

In a four-week repeated-dose toxicity study in rhesus monkeys with a four-week recovery period, conducted concurrently with safety pharmacology studies on the central nervous system (CNS), cardiovascular system (CVS), and respiratory system (RS) via subcutaneous injection of the AB8D monoclonal antibody injection, the AB8D monoclonal antibody injection showed no significant effects on the CNS, CVS, or RS of rhesus monkeys, and no local irritation at the administration site was observed.

In a dose-range finding and maximum tolerated dose (MTD) study in rhesus monkeys receiving subcutaneous injections of the AB8D monoclonal antibody drug substance, all animals survived until the scheduled necropsy. Female animals in the test article groups exhibited intermittent watery/loose stools and body weight loss. At the end of the observation period, prolonged activated partial thromboplastin time (APTT), increased serum blood urea nitrogen (BUN) and creatinine (CREA), and decreased sodium (Na) and chloride (Cl) levels were observed. Histopathological examination revealed mild cardiomyocyte degeneration in the heart and slight macrophage aggregation in the lungs. Due to the limited number of animals, the relationship of these abnormal findings to the test article could not be determined.

In the four-week repeated-dose toxicity study with a four-week recovery period in rhesus monkeys receiving subcutaneous injections of the AB8D monoclonal antibody injection, the observed changes included: compared to the vehicle control group, a decrease in plasma fibrinogen was observed in both sexes from 168 hours after the first dose until the end of the recovery period (Day 57) at doses ≥10 mg/kg. At doses ≥10 mg/kg, an increase or an upward trend in D-dimer levels was observed in both sexes from 24 hours after the first dose during the dosing period until the end of the dosing period (Day 29); at doses ≥30 mg/kg, an upward trend in D-dimer was observed at the end of the recovery period (Day 57). Histopathological examination revealed no thrombosis during or after the study period at doses of 10 and 30 mg/kg, and no test article-related toxic changes were observed.

In the in vitro hemolysis test of the AB8D monoclonal antibody injection, the AB8D monoclonal antibody injection did not cause hemolysis or agglutination of rabbit red blood cells in vitro, indicating its suitability for subcutaneous administration.

In the immunotoxicity evaluation conducted concurrently with the four-week repeated-dose toxicity study with a four-week recovery period in rhesus monkeys receiving subcutaneous injections of the AB8D monoclonal antibody injection, no abnormal changes in immunotoxicity evaluation indicators related to the test article were observed. This specifically includes hematological white blood cell indices; serum biochemical albumin and globulin levels; lymphocyte immunophenotyping (CD3+CD4+, CD3+CD8+, CD3+CD4+/CD3+CD8+ ratio, CD20+, CD16+); immunoglobulins and complement (IgA, IgG, IgM, C3, C4); immune organ weights (spleen and thymus); and gross anatomical observations and histopathological examinations of the spleen, thymus, lymph nodes (draining inguinal and mesenteric lymph nodes), and bone marrow.

In the cytokine release study following incubation of the AB8D monoclonal antibody injection with human peripheral blood mononuclear cells (PBMCs), the AB8D monoclonal antibody injection did not stimulate the release of IL-2, IL-4, IL-6, IL-10, TNF-α, or IFN-γ from PBMCs.

### Example 17: Pharmacokinetic Study of Anti-Human TFPI Antibodies in Rhesus Monkeys

Rhesus monkeys were randomly divided into two groups (6 animals per group, half male and half female). They received a single subcutaneous dose of the AB8D humanized antibody at 15 mg/kg or 50 mg/kg, respectively. The analyzed pharmacokinetic parameters are shown in Table 29. After a subcutaneous dose of 50 mg/kg, the half-life of the AB8D humanized antibody reached 136 hours.

**Table 29. Pharmacokinetic Parameters in Rhesus Monkeys**

| **Administration method** | **Subcutaneous injection** | **Subcutaneous injection** |
|---|---|---|
| **Dose (mg/kg)** | 15 | 50 |
| **Sample** | Plasma | Plasma |

| **PK Parameters:** | | |
|---|---|---|
| AUC_{INF obs}(h*µg/mL) | 27100±5280 | 123000±13900 |
| AUCₗₐₛₜ(h*µg/mL) | 26900±5410 | 123000±13900 |
| C₀(µg/mL) | - | - |
| Cₘₐₓ(µg/mL) | 137±16.7 | 523±26.9 |
| T_{1/2_z(}h) | 37.9±7.58 | 136±42.5 |
| Tₘₐₓ(h) | 36.0±36.0 | 36.0±24.0 |
| MRT_{INF_obs}(h) | 129±9.84 | 179±16.5 |
| MRTₗₐₛₜ(h) | 127±12.0 | 177±16.8 |
| Cl_{_F_obs}(mL/h/kg) | 0.571±0.104 | 0.410±0.0484 |
| Cl_{_obs}(mL/h/kg) | - | - |
| V_{z_F_obs}(mL/kg) | 31.7±10.7 | 79.3±22.4 |
| V_{z_obs}(mL/kg) | - | - |
| F(%) | 104 | - |

### Example 18: Safety Evaluation of Anti-Human TFPI Antibodies in Healthy Chinese Adult Subjects

In healthy subjects receiving a single dose of AB8D monoclonal antibody ranging from 30 to 200 mg, the incidence of adverse events showed a certain dose-dependency. The majority of adverse events were of Grade 1 severity (according to CTCAE v5.0 grading), with only one Grade II adverse event occurring, which was judged by the investigator to be unrelated to the investigational product. All adverse events were transient, with outcomes of complete recovery. No serious adverse events, adverse events leading to discontinuation, or deaths were reported. No clear, meaningful trends were observed in important safety laboratory parameters for all subjects. No other potentially significant safety concerns related to the AB8D monoclonal antibody were reported. Healthy subjects could tolerate a single subcutaneous injection of AB8D monoclonal antibody at doses of 10-200 mg, with an overall favorable safety and tolerability profile.

Globally, several drugs targeting the same pathway (anti-TFPI monoclonal antibodies) are under development for the treatment of Hemophilia A and B. Only partial data for Concizumab and Marstacimab have been disclosed. Concizumab has been approved in regions such as Canada, Japan, and Switzerland, while Marstacimab's highest global development stage is Phase III clinical trials. Adverse events reported with Concizumab in healthy subjects were mild or moderate in severity, with the most common adverse reactions being headache, common cold, fatigue, or influenza. Adverse events reported with Marstacimab in healthy subjects were mild or moderate in severity, with the most common adverse events including headache, fatigue, and local or systemic pain/discomfort. In the Phase I clinical trial of the AB8D monoclonal antibody, all reported adverse events were mild in severity. Headache, fatigue, or systemic pain/discomfort were not reported, indicating a potentially superior safety profile compared to similar agents in its class.

### Example 19: Clinical Pharmacokinetic Study of Anti-Human TFPI Antibody Following a Single Dose in Healthy Chinese Adults

An ELISA method was used to quantify the concentration of active AB8D in human plasma. Human TFPI/His (purchased from ACRO Biosystem, Cat. No. TFI-H5226) was diluted to 0.1 µg/ml in 1× PBS buffer and added to a 96-well ELISA plate at 100 µl per well, then incubated at 4°C for 16-20 hours. The PBS buffer was discarded from the plate, and 200 µl per well of PBST/1.5% BSA was added for blocking, followed by incubation at room temperature for 1 hour. After washing the plate 3 times with PBST buffer (pH 7.4, PBS containing 0.05% Tween 20), the test plasma samples were added at 100 µl per well and incubated at room temperature for 1.0 hour. Following another 3 washes with PBST, 100 µl per well of a 10 µg/ml biotinylated mouse anti-AB8D idiotype antibody (produced by Ampsource Biopharma, AP651651) was added and incubated at room temperature for 1 hour. The plate was washed 3 times with PBST, then 100 µl per well of a 1:5000 dilution of SA-Avidin-HRP was added and incubated at room temperature for 1 hour. After a final 3 washes with PBST, 100 µl per well of TMB substrate was added and incubated at room temperature for 5-10 minutes. The reaction was stopped by adding 50 µl per well of 0.2 M sulfuric acid. Absorbance was read at dual wavelengths of 450/620 nm using a microplate reader.

Regarding pharmacokinetics, the AB8D humanized antibody exhibited nonlinear pharmacokinetic characteristics within the tested dose range, consistent with target-mediated drug disposition (TMDD) effects. The pharmacokinetic parameters in healthy subjects are shown in Table 30. Following subcutaneous administration in healthy subjects, the time to peak concentration (Tmax) ranged from 31 to 36 hours; the mean half-life (t1/2) ranged from 104 to 145 hours, and the half-life increased with higher doses. Compared to drugs targeting the same pathway, AB8D demonstrated a more rapid Tmax and a longer half-life, both of which are pharmacokinetic features associated with TMDD.

**Table 30. Pharmacokinetic Parameters in Healthy Subjects**

| **Dose Group** | **S1 Group (30mg)** | **S2 Group (100mg)** |
|---|---|---|
| **Sample Size (n)** | N=4 | N=8 |
| Tmax, h | 31.00(10~96) | 36.00(4~48) |
| Cmax, ng/mL(CV) | 74.40±9.52 (12.80) | 1,935.63±2179.05 (112.58) |
| AUC0-t, h*ng/mL(CV) | 8,691.65±1293.08 (14.88) | 108,490.75±92644.93 (85.39) |
| AUC0-∞, h*ng/mL(CV) | 13,356.91±1896.87 (14.20) | 116,282.68±92218.51 (79.31) |
| t1/2, h | 116,282.68±92218.51 (79.31) | 145.85±35.03 (24.02) |
| MPT0-t,h | 80.8 | 80.67 |

### Example 20: Clinical Pharmacodynamic Study of Anti-Human TFPI Antibody Following a Single Dose in Healthy Chinese Adults

A sandwich ELISA method was used to quantify the concentration of TFPI in human plasma. AB8D was diluted to 5 µg/ml in 1× PBS buffer and added to a 96-well ELISA plate at 100 µl per well, then incubated at 4°C for 16-20 hours. The PBS buffer was discarded from the plate, and 200 µl per well of PBST/1.5% BSA was added for blocking, followed by incubation at room temperature for 1 hour. After washing the plate 3 times with PBST buffer (pH 7.4, PBS containing 0.05% Tween 20), the test plasma samples were added at 100 µl per well and incubated at room temperature for 1.0 hour. Following another 3 washes with PBST, 100 µl per well of a 0.1 µg/ml rabbit anti-human TFPI antibody (purchased from Abcam, ab274436) was added and incubated at room temperature for 1 hour. The plate was washed 3 times with PBST, then 100 µl per well of a 1:80,000 dilution of Peroxidase-conjugated AffiniPure Donkey Anti-Rabbit IgG (H+L) (purchased from Jackson ImmunoResearch) was added and incubated at room temperature for 1 hour. After a final 3 washes with PBST, 100 µl per well of TMB substrate was added and incubated at room temperature for 5-10 minutes. The reaction was stopped by adding 50 µl per well of 0.2 M sulfuric acid. Absorbance was read at dual wavelengths of 450/620 nm using a microplate reader.

A reduction in free TFPI levels was observed across all AB8D monoclonal antibody dose groups (30 mg to 200 mg), with a dose-dependent decrease. Free TFPI levels decreased rapidly within 8 hours after the first administration of AB8D. In the low-dose group (30 mg), TFPI returned to baseline levels around Day 14, while in the high-dose group (200 mg), it returned to baseline around Day 21.

While preferred embodiments of the invention have been illustrated and described, it should be understood that those skilled in the art may make various changes in light of the teachings herein, without departing from the scope of the invention.

All documents mentioned in this invention are incorporated herein by reference, as if each document were individually incorporated by reference. Furthermore, it should be understood that after reading the above teachings of the present invention, those skilled in the art may make various modifications or changes, all of which equivalent forms also fall within the scope defined by the appended claims of this application.

## Claims

1. An antibody or antigen-binding fragment thereof that binds to Tissue Factor Pathway Inhibitor (TFPI), wherein the antibody or antigen-binding fragment thereof does not interact with and/or block R107 in the TFPI within 5 Å.

2. The antibody or antigen-binding fragment thereof according to claim 1, wherein the antibody or antigen-binding fragment thereof interacts with and/or blocks at least one amino acid residue in the TFPI selected from Y109, I110, T111, R124, K126, and E138 at a distance of 5 Å or less.

3. The antibody or antigen-binding fragment thereof according to claim 2, wherein the antibody or antigen-binding fragment thereof interacts with and/or blocks at least one amino acid residue in the TFPI selected from T139, T119, E101, Y113, F114, N116, Q118, Q121, C122, E123, F125, and L140 at a distance of 5 Å or less.

4. The antibody or antigen-binding fragment thereof according to claim 2, wherein the antibody or antigen-binding fragment thereof interacts with and/or blocks at least one amino acid residue in the TFPI selected from R112, K93, K120, K126, and K144, preferably R124 and K126, at a distance of 5 Å or less.

5. The antibody or antigen-binding fragment thereof according to claim 1, **characterized in that** the amino acid sequence of the TFPI is as shown in SEQ ID NO: 1.

6. The antibody or antigen-binding fragment thereof according to claim 1, **characterized in that** the antibody or antigen-binding fragment thereof comprises a heavy chain variable region (VH) and a light chain variable region (VL), wherein,
(a) the heavy chain variable region, as defined by the Kabat numbering system, comprises at least one, two, or three CDRs selected from the group consisting of:
(1) HCDR1 having the amino acid sequence SYWMH;
(2) HCDR2 having the amino acid sequence EIKPDX₁GRIX₂YNEKFKT, wherein X₁ is selected from Q, H, or K, and X₂ is selected from N, D, or H; and
(3) HCDR3 having the amino acid sequence SSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or,
the light chain variable region comprises at least one, two, or three CDRsselected from the group consisting of:
(4) LCDR1 having the amino acid sequence SASSSVSSSYLY;
(5) LCDR2 having the amino acid sequence GTSILAS; and
(6) LCDR3 having the amino acid sequence HQWX₇X₈YPFT, wherein X₇ is selected from S or T, and X₈ is selected from H, Y, or F;
or,
(b) the heavy chain variable region, as defined by the IMGT numbering system, comprises at least one, two, or three CDRs selected from the group consisting of:
(1) HCDR1 having the amino acid sequence GYTFTSYW;
(2) HCDR2 having the amino acid sequence IKPDX₁GRI, wherein X₁ is selected from Q, H, or K; and
(3) HCDR3 having the amino acid sequence ARSSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or,
the light chain variable region comprises at least one, two, or three CDRs selected from the group consisting of:
(4) LCDR1 having the amino acid sequence SSVSSSY;
(5) LCDR2 having the amino acid sequence GTS; and
(6) LCDR3 having the amino acid sequence HQWX₇X₈YPFT, wherein X₇ is selected from S or T, and X₈ is selected from H, Y, or F.

7. The antibody or antigen-binding fragment thereof according to claim 6, **characterized in that** the antibody or antigen-binding fragment thereof comprises 3 VH CDRs and 3 VL CDRs selected from the group consisting of:
(1) HCDR1, HCDR2, HCDR3, LCDR1, LCDR2, and LCDR3 having the sequences as shown in SEQ ID NOs: 46, 47, 48, 49, 50, and 51, respectively, or sequences having one or several amino acid substitutions, deletions, or additions (e.g., 1, 2, or 3 substitutions, deletions, or additions) compared to any of the aforementioned sequences;
(2) HCDR1, HCDR2, HCDR3, LCDR1, LCDR2, and LCDR3 having the sequences as shown in SEQ ID NOs: 46, 47, 56, 49, 50, and 51, respectively, or sequences having one or several amino acid substitutions, deletions, or additions (e.g., 1, 2, or 3 substitutions, deletions, or additions) compared to any of the aforementioned sequences;
(3) HCDR1, HCDR2, HCDR3, LCDR1, LCDR2, and LCDR3 having the sequences as shown in SEQ ID NOs: 46, 47, 58, 49, 50, and 51, respectively, or sequences having one or several amino acid substitutions, deletions, or additions (e.g., 1, 2, or 3 substitutions, deletions, or additions) compared to any of the aforementioned sequences;
(4) HCDR1, HCDR2, HCDR3, LCDR1, LCDR2, and LCDR3 having the sequences as shown in SEQ ID NOs: 46, 60, 48, 49, 50, and 51, respectively, or sequences having one or several amino acid substitutions, deletions, or additions (e.g., 1, 2, or 3 substitutions, deletions, or additions) compared to any of the aforementioned sequences;
(5) HCDR1, HCDR2, HCDR3, LCDR1, LCDR2, and LCDR3 having the sequences as shown in SEQ ID NOs: 46, 61, 48, 49, 50, and 51, respectively, or sequences having one or several amino acid substitutions, deletions, or additions (e.g., 1, 2, or 3 substitutions, deletions, or additions) compared to any of the aforementioned sequences;
(6) HCDR1, HCDR2, HCDR3, LCDR1, LCDR2, and LCDR3 having the sequences as shown in SEQ ID NOs: 46, 63, 48, 49, 50, and 51, respectively, or sequences having one or several amino acid substitutions, deletions, or additions (e.g., 1, 2, or 3 substitutions, deletions, or additions) compared to any of the aforementioned sequences;
(7) HCDR1, HCDR2, HCDR3, LCDR1, LCDR2, and LCDR3 having the sequences as shown in SEQ ID NOs: 46, 65, 48, 49, 50, and 51, respectively, or sequences having one or several amino acid substitutions, deletions, or additions (e.g., 1, 2, or 3 substitutions, deletions, or additions) compared to any of the aforementioned sequences;
(8) HCDR1, HCDR2, HCDR3, LCDR1, LCDR2, and LCDR3 having the sequences as shown in SEQ ID NOs: 46, 47, 48, 49, 50, and 66, respectively, or sequences having one or several amino acid substitutions, deletions, or additions (e.g., 1, 2, or 3 substitutions, deletions, or additions) compared to any of the aforementioned sequences;
(9) HCDR1, HCDR2, HCDR3, LCDR1, LCDR2, and LCDR3 having the sequences as shown in SEQ ID NOs: 46, 47, 48, 49, 50, and 67, respectively, or sequences having one or several amino acid substitutions, deletions, or additions (e.g., 1, 2, or 3 substitutions, deletions, or additions) compared to any of the aforementioned sequences;
(10) HCDR1, HCDR2, HCDR3, LCDR1, LCDR2, and LCDR3 having the sequences as shown in SEQ ID NOs: 46, 47, 48, 49, 50, and 68, respectively, or sequences having one or several amino acid substitutions, deletions, or additions (e.g., 1, 2, or 3 substitutions, deletions, or additions) compared to any of the aforementioned sequences;
(11) HCDR1, HCDR2, HCDR3, LCDR1, LCDR2, and LCDR3 having the sequences as shown in SEQ ID NOs: 46, 63, 58, 49, 50, and 51, respectively, or sequences having one or several amino acid substitutions, deletions, or additions (e.g., 1, 2, or 3 substitutions, deletions, or additions) compared to any of the aforementioned sequences;
(12) HCDR1, HCDR2, HCDR3, LCDR1, LCDR2, and LCDR3 having the sequences as shown in SEQ ID NOs: 46, 47, 69, 49, 50, and 51, respectively, or sequences having one or several amino acid substitutions, deletions, or additions (e.g., 1, 2, or 3 substitutions, deletions, or additions) compared to any of the aforementioned sequences;
(13) HCDR1, HCDR2, HCDR3, LCDR1, LCDR2, and LCDR3 having the sequences as shown in SEQ ID NOs: 46, 47, 71, 49, 50, and 51, respectively, or sequences having one or several amino acid substitutions, deletions, or additions (e.g., 1, 2, or 3 substitutions, deletions, or additions) compared to any of the aforementioned sequences;
(14) HCDR1, HCDR2, HCDR3, LCDR1, LCDR2, and LCDR3 having the sequences as shown in SEQ ID NOs: 46, 47, 73, 49, 50, and 51, respectively, or sequences having one or several amino acid substitutions, deletions, or additions (e.g., 1, 2, or 3 substitutions, deletions, or additions) compared to any of the aforementioned sequences;
(15) HCDR1, HCDR2, HCDR3, LCDR1, LCDR2, and LCDR3 having the sequences as shown in SEQ ID NOs: 46, 47, 75, 49, 50, and 51, respectively, or sequences having one or several amino acid substitutions, deletions, or additions (e.g., 1, 2, or 3 substitutions, deletions, or additions) compared to any of the aforementioned sequences;
(16) HCDR1, HCDR2, HCDR3, LCDR1, LCDR2, and LCDR3 having the sequences as shown in SEQ ID NOs: 46, 47, 77, 49, 50, and 51, respectively, or sequences having one or several amino acid substitutions, deletions, or additions (e.g., 1, 2, or 3 substitutions, deletions, or additions) compared to any of the aforementioned sequences;
(17) HCDR1, HCDR2, HCDR3, LCDR1, LCDR2, and LCDR3 having the sequences as shown in SEQ ID NOs: 46, 47, 79, 49, 50, and 51, respectively, or sequences having one or several amino acid substitutions, deletions, or additions (e.g., 1, 2, or 3 substitutions, deletions, or additions) compared to any of the aforementioned sequences;
(18) HCDR1, HCDR2, HCDR3, LCDR1, LCDR2, and LCDR3 having the sequences as shown in SEQ ID NOs: 46, 47, 81, 49, 50, and 51, respectively, or sequences having one or several amino acid substitutions, deletions, or additions (e.g., 1, 2, or 3 substitutions, deletions, or additions) compared to any of the aforementioned sequences;
(19) HCDR1, HCDR2, HCDR3, LCDR1, LCDR2, and LCDR3 having the sequences as shown in SEQ ID NOs: 46, 47, 83, 49, 50, and 51, respectively, or sequences having one or several amino acid substitutions, deletions, or additions (e.g., 1, 2, or 3 substitutions, deletions, or additions) compared to any of the aforementioned sequences;
(20) HCDR1, HCDR2, HCDR3, LCDR1, LCDR2, and LCDR3 having the sequences as shown in SEQ ID NOs: 46, 47, 85, 49, 50, and 51, respectively, or sequences having one or several amino acid substitutions, deletions, or additions (e.g., 1, 2, or 3 substitutions, deletions, or additions) compared to any of the aforementioned sequences;
(21) HCDR1, HCDR2, HCDR3, LCDR1, LCDR2, and LCDR3 having the sequences as shown in SEQ ID NOs: 46, 47, 87, 49, 50, and 51, respectively, or sequences having one or several amino acid substitutions, deletions, or additions (e.g., 1, 2, or 3 substitutions, deletions, or additions) compared to any of the aforementioned sequences;
(22) HCDR1, HCDR2, HCDR3, LCDR1, LCDR2, and LCDR3 having the sequences as shown in SEQ ID NOs: 46, 47, 89, 49, 50, and 51, respectively, or sequences having one or several amino acid substitutions, deletions, or additions (e.g., 1, 2, or 3 substitutions, deletions, or additions) compared to any of the aforementioned sequences;
(23) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(24) HCDR1, HCDR2, HCDR3, LCDR1, LCDR2, and LCDR3 having the sequences as shown in SEQ ID NOs: 52, 53, 57, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(25) HCDR1, HCDR2, HCDR3, LCDR1, LCDR2, and LCDR3 having the sequences as shown in SEQ ID NOs: 52, 53, 59, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(26) HCDR1, HCDR2, HCDR3, LCDR1, LCDR2, and LCDR3 having the sequences as shown in SEQ ID NOs: 52, 62, 54, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(27) HCDR1, HCDR2, HCDR3, LCDR1, LCDR2, and LCDR3 having the sequences as shown in SEQ ID NOs: 52, 65, 54, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(28) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 66, respectively, or sequences having one or several amino acid substitutions, deletions, or additions (e.g., 1, 2, or 3 substitutions, deletions, or additions) compared to any of the aforementioned sequences;
(29) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 67, respectively, or sequences having one or several amino acid substitutions, deletions, or additions (e.g., 1, 2, or 3 substitutions, deletions, or additions) compared to any of the aforementioned sequences;
(30) HCDR1, HCDR2, HCDR3, LCDR1, LCDR2, and LCDR3 having the sequences as shown in SEQ ID NOs: 52, 53, 54, 55, Gly-Thr-Ser, or SEQ ID NO: 68, respectively, or sequences having one or several amino acid substitutions, deletions, or additions (e.g., 1, 2, or 3 substitutions, deletions, or additions) compared to any of the aforementioned sequences;
(31) HCDR1, HCDR2, HCDR3, LCDR1, LCDR2, and LCDR3 having the sequences as shown in SEQ ID NOs: 52, 64, 59, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(32) HCDR1, HCDR2, HCDR3, LCDR1, LCDR2, and LCDR3 having the sequences as shown in SEQ ID NOs: 52, 53, 70, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(33) HCDR1, HCDR2, HCDR3, LCDR1, LCDR2, and LCDR3 having the sequences as shown in SEQ ID NOs: 52, 53, 72, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(34) HCDR1, HCDR2, HCDR3, LCDR1, LCDR2, and LCDR3 having the sequences as shown in SEQ ID NOs: 52, 53, 74, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(35) HCDR1, HCDR2, HCDR3, LCDR1, LCDR2, and LCDR3 having the sequences as shown in SEQ ID NOs: 52, 53, 76, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(36) HCDR1, HCDR2, HCDR3, LCDR1, LCDR2, and LCDR3 having the sequences as shown in SEQ ID NOs: 52, 53, 78, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(37) HCDR1, HCDR2, HCDR3, LCDR1, LCDR2, and LCDR3 having the sequences as shown in SEQ ID NOs: 52, 53, 80, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(38) HCDR1, HCDR2, HCDR3, LCDR1, LCDR2, and LCDR3 having the sequences as shown in SEQ ID NOs: 52, 53, 82, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(39) HCDR1, HCDR2, HCDR3, LCDR1, LCDR2, and LCDR3 having the sequences as shown in SEQ ID NOs: 52, 53, 84, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(40) HCDR1, HCDR2, HCDR3, LCDR1, LCDR2, and LCDR3 having the sequences as shown in SEQ ID NOs: 52, 53, 86, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(41) HCDR1, HCDR2, HCDR3, LCDR1, LCDR2, and LCDR3 having the sequences as shown in SEQ ID NOs: 52, 53, 88, 55 Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences;
(42) HCDR1, HCDR2, HCDR3, LCDR1, LCDR2, and LCDR3 having the sequences as shown in SEQ ID NOs: 52, 53, 90, 55, Gly-Thr-Ser, or SEQ ID NO: 51, respectively, or sequences having one or several amino acid substitutions, deletions, or additions (e.g., 1, 2, or 3 substitutions, deletions, or additions) compared to any of the aforementioned sequences.

8. The antibody or antigen-binding fragment thereof according to claim 6, **characterized in that** the antibody or antigen-binding fragment thereof is humanized.

9. The antibody or antigen-binding fragment thereof according to claim 8, **characterized in that** the antibody or antigen-binding fragment thereof comprises VH and VL sequence combinations selected from the following:
(1) a VH domain comprising the amino acid sequence as shown in SEQ ID NO:2, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:3, or a sequence substantially identical thereto (e.g., at least 80%, 85%, 90%, 92%, 95%, 97%, 98%, 99% or higher identity, or having one or more amino acid substitutions (e.g., conservative substitutions));
(2) a VH domain comprising the amino acid sequence as shown in SEQ ID NO:4, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:5, or a sequence substantially identical thereto (e.g., at least 80%, 85%, 90%, 92%, 95%, 97%, 98%, 99% or higher identity, or having one or more amino acid substitutions (e.g., conservative substitutions));
(3) a VH domain comprising the amino acid sequence as shown in SEQ ID NO:6, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:7, or a sequence substantially identical thereto (e.g., at least 80%, 85%, 90%, 92%, 95%, 97%, 98%, 99% or higher identity, or having one or more amino acid substitutions (e.g., conservative substitutions));
(4) a VH domain comprising the amino acid sequence as shown in SEQ ID NO:8, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:9, or a sequence substantially identical thereto (e.g., at least 80%, 85%, 90%, 92%, 95%, 97%, 98%, 99% or higher identity, or having one or more amino acid substitutions (e.g., conservative substitutions));
(5) a VH domain comprising the amino acid sequence as shown in SEQ ID NO:10, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:11, or a sequence substantially identical thereto (e.g., at least 80%, 85%, 90%, 92%, 95%, 97%, 98%, 99% or higher identity, or having one or more amino acid substitutions (e.g., conservative substitutions));
(6) a VH domain comprising the amino acid sequence as shown in SEQ ID NO:12, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:13, or a sequence substantially identical thereto (e.g., at least 80%, 85%, 90%, 92%, 95%, 97%, 98%, 99% or higher identity, or having one or more amino acid substitutions (e.g., conservative substitutions));
(7) a VH domain comprising the amino acid sequence as shown in SEQ ID NO:14, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:15, or a sequence substantially identical thereto (e.g., at least 80%, 85%, 90%, 92%, 95%, 97%, 98%, 99% or higher identity, or having one or more amino acid substitutions (e.g., conservative substitutions));
(8) a VH domain comprising the amino acid sequence as shown in SEQ ID NO:16, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:17, or a sequence substantially identical thereto (e.g., at least 80%, 85%, 90%, 92%, 95%, 97%, 98%, 99% or higher identity, or having one or more amino acid substitutions (e.g., conservative substitutions));
(9) a VH domain comprising the amino acid sequence as shown in SEQ ID NO:18, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:19, or a sequence substantially identical thereto (e.g., at least 80%, 85%, 90%, 92%, 95%, 97%, 98%, 99% or higher identity, or having one or more amino acid substitutions (e.g., conservative substitutions));
(10) a VH domain comprising the amino acid sequence as shown in SEQ ID NO:20, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:21, or a sequence substantially identical thereto (e.g., at least 80%, 85%, 90%, 92%, 95%, 97%, 98%, 99% or higher identity, or having one or more amino acid substitutions (e.g., conservative substitutions));
(11) a VH domain comprising the amino acid sequence as shown in SEQ ID NO:22, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:23, or a sequence substantially identical thereto (e.g., at least 80%, 85%, 90%, 92%, 95%, 97%, 98%, 99% or higher identity, or having one or more amino acid substitutions (e.g., conservative substitutions));
(12) a VH domain comprising the amino acid sequence as shown in SEQ ID NO:24, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:25, or a sequence substantially identical thereto (e.g., at least 80%, 85%, 90%, 92%, 95%, 97%, 98%, 99% or higher identity, or having one or more amino acid substitutions (e.g., conservative substitutions));
(13) a VH domain comprising the amino acid sequence as shown in SEQ ID NO:26, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:27, or a sequence substantially identical thereto (e.g., at least 80%, 85%, 90%, 92%, 95%, 97%, 98%, 99% or higher identity, or having one or more amino acid substitutions (e.g., conservative substitutions));
(14) a VH domain comprising the amino acid sequence as shown in SEQ ID NO:28, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:29, or a sequence substantially identical thereto (e.g., at least 80%, 85%, 90%, 92%, 95%, 97%, 98%, 99% or higher identity, or having one or more amino acid substitutions (e.g., conservative substitutions));
(15) a VH domain comprising the amino acid sequence as shown in SEQ ID NO:30, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:31, or a sequence substantially identical thereto (e.g., at least 80%, 85%, 90%, 92%, 95%, 97%, 98%, 99% or higher identity, or having one or more amino acid substitutions (e.g., conservative substitutions));
(16) a VH domain comprising the amino acid sequence as shown in SEQ ID NO:32, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:33, or a sequence substantially identical thereto (e.g., at least 80%, 85%, 90%, 92%, 95%, 97%, 98%, 99% or higher identity, or having one or more amino acid substitutions (e.g., conservative substitutions));
(17) a VH domain comprising the amino acid sequence as shown in SEQ ID NO:34, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:35, or a sequence substantially identical thereto (e.g., at least 80%, 85%, 90%, 92%, 95%, 97%, 98%, 99% or higher identity, or having one or more amino acid substitutions (e.g., conservative substitutions));
(18) a VH domain comprising the amino acid sequence as shown in SEQ ID NO:36, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:37, or a sequence substantially identical thereto (e.g., at least 80%, 85%, 90%, 92%, 95%, 97%, 98%, 99% or higher identity, or having one or more amino acid substitutions (e.g., conservative substitutions));
(19) a VH domain comprising the amino acid sequence as shown in SEQ ID NO:38, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:39, or a sequence substantially identical thereto (e.g., at least 80%, 85%, 90%, 92%, 95%, 97%, 98%, 99% or higher identity, or having one or more amino acid substitutions (e.g., conservative substitutions));
(20) a VH domain comprising the amino acid sequence as shown in SEQ ID NO:40, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:41, or a sequence substantially identical thereto (e.g., at least 80%, 85%, 90%, 92%, 95%, 97%, 98%, 99% or higher identity, or having one or more amino acid substitutions (e.g., conservative substitutions));
(21) a VH domain comprising the amino acid sequence as shown in SEQ ID NO:42, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:43, or a sequence substantially identical thereto (e.g., at least 80%, 85%, 90%, 92%, 95%, 97%, 98%, 99% or higher identity, or having one or more amino acid substitutions (e.g., conservative substitutions));
(22) a VH domain comprising the amino acid sequence as shown in SEQ ID NO:44, or a sequence substantially identical thereto (e.g., at least 80%, 85%, 90%, 92%, 95%, 97%, 98%, 99% or higher identity, or having one or more amino acid substitutions (e.g., conservative substitutions)); and a VL domain comprising the amino acid sequence as shown in SEQ ID NO:45, or a sequence substantially identical thereto (e.g., at least 80%, 85%, 90%, 92%, 95%, 97%, 98%, 99% or higher identity, or having one or more amino acid substitutions (e.g., conservative substitutions)).

10. The antibody or antigen-binding fragment thereof according to claim 1, **characterized in that** it is a full-length antibody, Fab, Fab', (Fab')₂, Fd, Fv, dAb, scFv or scFv-scFv.

11. The antibody or antigen-binding fragment thereof according to claim 1, **characterized in that** the antibody comprises a heavy chain constant region and a light chain constant region derived from a human immunoglobulin; preferably, the heavy chain constant region is selected from the heavy chain constant regions of human IgG1, IgG2, IgG3, and IgG4; and, the heavy chain constant region has a natural sequence or a sequence having one or more amino acid substitutions, deletions, or additions compared to the natural sequence from which it is derived; and the light chain constant region is preferably the constant region of a human kappa light chain.

12. The antibody or antigen-binding fragment thereof according to claim 11, **characterized in that** the antibody comprises a heavy chain constant region of human IgG4, and the heavy chain constant region contains the amino acid mutation S228P.

13. A bispecific antibody or antigen-binding portion thereof, comprising the antibody or antigen-binding portion thereof according to any one of claims 1-12 linked to a second antibody or antigen-binding portion thereof.

14. The bispecific antibody or antigen-binding portion thereof according to claim 13, **characterized in that** the second antibody or antigen-binding portion thereof binds to TFPI.

15. A DNA molecule encoding the antibody or antigen-binding fragment thereof according to any one of claims 1-14.

16. A vector comprising the DNA molecule according to claim 15.

17. A host cell comprising the vector according to claim 16, preferably the host cell is a prokaryotic cell, yeast or mammalian cell, more preferably a CHO cell.

18. A pharmaceutical composition comprising the antibody or antigen-binding fragment thereof according to any one of claims 1-14, and a pharmaceutically acceptable excipient, carrier or diluent.

19. Use of the antibody or antigen-binding fragment thereof according to any one of claims 1-14, or the pharmaceutical composition according to claim 18, in the manufacture of a medicament for preventing or treating a disease or condition associated with hereditary or acquired coagulation factor deficiency.

20. A method for preventing or treating a disease or condition associated with hereditary or acquired coagulation factor deficiency, comprising administering an effective amount of the antibody or antigen-binding fragment thereof according to any one of claims 1-14, or the pharmaceutical composition according to claim 18, to a patient in need thereof.

21. The use according to claim 19 or the method according to claim 20, **characterized in that** the disease is hemophilia.

22. Use of the antibody or antigen-binding fragment thereof according to any one of claims 1-14, or the pharmaceutical composition according to claim 18, in the manufacture of a medicament for preventing or treating a hereditary coagulation disorder.

23. A method for preventing or treating a hereditary coagulation disorder, comprising administering an effective amount of the antibody or antigen-binding fragment thereof according to any one of claims 1-14, or the pharmaceutical composition according to claim 18, to a patient in need thereof.

24. The use according to claim 22 or the method according to claim 23, **characterized in that** the disease is Glanzmann Thrombasthenia or Bernard-Soulier Syndrome.
